# EUROPÄISCHE PATENTANMELDUNG

(11) **EP 4 439 574 A1**
(43) Veröffentlichungstag der Anmeldung: **02.10.2024**
(21) Anmeldenummer: 24164647.0
(22) Anmeldetag: 19.03.2024
(51) Int. Cl.: G16H 10/60, G16H 50/70

(54) **VERFAHREN ZUM ERSTELLEN UND ABGLEICHEN VON PERSÖNLICHKEITSPROFILEN SOWIE ENTSPRECHENDE DATENBANK, COMPUTERPROGRAMMPRODUKT, BENUTZERSCHNITTSTELLE UND VERWENDUNG**

(30) Priorität: 23.03.2023 DE 102023107344; 23.03.2023 DE 202023101465 U
(71) Anmelder: Qonhuman GmbH, 60323 Frankfurt am Main (DE)
(72) Erfinder: Fechtner, Lisa, 55283 Nierstein (DE); Mahrenholz, Mandy-Marie, 65929 Frankfurt am Main (DE)
(74) Vertreter: Tanner, Lukas

(57) **Zusammenfassung**

Bei der Bestimmung eines Persönlichkeitsprofils besteht Interesse an einer möglichst breiten Datengrundlage, insbesondere um eine möglichst belastbare Charakterisierung vornehmen zu können. Bereitgestellt wird ein Verfahren zum Erstellen und Abgleichen von Persönlichkeitsprofilen, wobei dafür eine Datengrundlage basierend auf zumindest den folgenden individuenspezifischen Kennwerten erstellt oder abgerufen sowie ausgewertet wird: Geburtsdatum, Geburtszeit, Geburtsort, wahlweise auch Name und/oder Profilbild; Erfindungsgemäß wird die Datengrundlage ergänzt, indem eine Mehrzahl von eigenständigen Charakterprofildatensätzen zum individuenspezifischen Erstellen/Generieren eines charakterprofildatensatzübergreifenden Persönlichkeitsprofils herangezogen werden, insbesondere wenigstens fünf eigenständige Charakterprofildatensätze, insbesondere Charakterprofildatensätze betreffend die folgenden Charakterprofile: Human Design-Profil(daten), Genekeys-Profil(daten), Numerologie-Profil(daten), Bazi Suan Ming-Profil(daten), Astrologie-Profil(daten). Hierdurch kann der Umfang der auswertbaren Daten erweitert werden, und das Abgleichen kann hinsichtlich zahlreicher Kriterien und bei hoher Datengüte erfolgen. Die Erfindung betrifft ferner entsprechende Vorrichtungen und Verwendungen.

## Beschreibung

### TECHNISCHES GEBIET

Die vorliegende Erfindung betrifft ein Verfahren zum computergestützten/-implementierten Erstellen eines Persönlichkeitsprofils eines Individuums und zum Abgleichen des Persönlichkeitsprofils mit dem Persönlichkeitsprofil wenigstens eines weiteren Individuums. Ferner betrifft die vorliegende Erfindung eine entsprechend ausgestaltete Datenbank und ein entsprechend ausgestaltetes Computerprogrammprodukt, insbesondere auch eine für ein solches Verfahren eingerichtete Benutzerschnittstelle. Nicht zuletzt betrifft die vorliegende Erfindung auch die Verwendung einer auf Codes mehrerer vorbekannter Charakterisierungs-Ansätze basierenden (Daten-)Matrix zum Abgleichen von Persönlichkeitsprofilen. Insbesondere betrifft die Erfindung Verfahren und Vorrichtungen gemäß dem Oberbegriff des jeweiligen unabhängigen Anspruchs.

### HINTERGRUND DER ERFINDUNG

Für die Charakterisierung der Persönlichkeit (bzw. der Charaktereigenschaften) einzelner Individuen können unterschiedliche Systeme oder Ansätze zu Grunde gelegt werden, insbesondere um darauf basierend eine Klassifizierung von Charaktermerkmalen standardisieren zu können. Gemäß dem Stand der Technik wird unterschieden zwischen mehreren Charakterisierungs-Ansätzen bzw. Bewertungssystemen, welche jeweils auch hinsichtlich bestimmter Zwecke oder Gewichtungen bevorzugt ausgewählt und einzeln herangezogen werden können, insbesondere zwischen den folgenden fünf Ansätze: Bazi Suan Ming-Ansatz, Human Design-Ansatz, Genekeys-Ansatz, Numerologie-Ansatz, Astrologie-Ansatz. Jeder dieser Ansätze kann dabei insbesondere zweckgebunden besonders vorteilhaft sein bzw. im Rahmen der vorliegenden Erfindung zielgerichtet ausgewertet werden.

Davon ausgehend besteht Interesse an einem verbesserten Konzept zum möglichst exakten und detailreichen Charakterisieren von Individuen sowie zum Differenzieren einer Vielzahl von Individuen voneinander insbesondere hinsichtlich einzelner Charaktermerkmale. Ein entsprechendes Verfahren soll möglichst auch computergestützt ausgeführt oder zumindest erleichtert werden können.

Beispielhaft kann die Veröffentlichung US 9,002,961 B1 genannt werden, welche die Verarbeitung von so genannten MBTI-Kennzahlen (Myer-Briggs Type Indicator), astrologischen Zeichen und Merkmalen für ein Zuordnen bzw. für ein kommunikatives Verknüpfen von Individuen beschreibt.

Die Veröffentlichung DE 21 2015 000 172 U1 beschreibt ein System zur Unterstützung bei Lebensentscheidungen im Zusammenhang mit einem Verfahren zum Zuordnen von Personen, bei welchem der Geburtstag bzw. die Geburtszeit von einer oder zwei Personen berücksichtigt werden kann, wobei über eine Benutzerschnittstelle eine Interaktion mit einer Datenbank ermöglicht wird.

Ausgehend vom Stand der Technik ist ein Bedarf an Verfahren und Vorrichtungen zu spüren, womit Persönlichkeitsprofile noch treffsicherer und detailreicher ermittelt und miteinander verglichen werden können, insbesondere auch bei möglichst einfacher Handhabung der Verarbeitung der damit verbundenen Daten. Nicht zuletzt besteht insbesondere hinsichtlich einer zielsicheren Verknüpfung von Individuen auch Interesse an einem möglichst viele Kriterien berücksichtigenden Werkzeug, welches insbesondere auch das Bilden von Gruppen miteinander harmonierender Individuen erleichtert.

### ZUSAMMENFASSUNG DER ERFINDUNG

Aufgabe ist, ein Verfahren und eine Vorrichtung bzw. ein Werkzeug zum Erstellen eines Persönlichkeitsprofils wenigstens eines Individuums und zum Abgleichen des Persönlichkeitsprofils mit dem jeweiligen Persönlichkeitsprofil wenigstens eines weiteren Individuums bereitzustellen, womit die jeweiligen Persönlichkeitseigenschaften möglichst treffend und detailliert bestimmt werden können und eine möglichst umfangreiche Charakterisierung der Persönlichkeit sichergestellt werden kann. Auch ist es Aufgabe, ein zumindest teilweise computerimplementiertes Verfahren zur Persönlichkeitsprofilbestimmung und diesbezüglichem Persönlichkeitsprofilabgleich derart auszugestalten, dass zunächst eine möglichst breite und belastbare Datengrundlage hoher Güte geschaffen werden kann und diese für einen Abgleich von Persönlichkeitsprofilen auf vorteilhaft flexible und individualisierbare Weise genutzt werden kann.

Diese Aufgabe wird durch ein Verfahren gemäß Anspruch 1 sowie durch eine Datenbank, durch ein Computerprogrammprodukt, durch eine Benutzerschnittstelle und durch Verwendungen jeweils gemäß dem nebengeordneten Anspruch gelöst. Vorteilhafte Weiterbildungen der Erfindung werden in den jeweiligen Unteransprüchen erläutert. Die Merkmale der im Folgenden beschriebenen Ausführungsbeispiele sind miteinander kombinierbar, sofern dies nicht explizit verneint ist.

Bereitgestellt wird ein Verfahren zum computergestützten/-implementierten Erstellen eines Persönlichkeitsprofils eines Individuums und zum Abgleichen des Persönlichkeitsprofils mit dem Persönlichkeitsprofil wenigstens eines weiteren Individuums, wobei eine Datengrundlage (Datenbasis) für die Erstellung des Persönlichkeitsprofils basierend auf zumindest den folgenden bevorzugt über eine Benutzerschnittstelle (Eingabemaske) manuell eingebbaren oder zumindest teilweise automatisiert aus einer Datenbank abrufbaren individuenspezifischen Kennwerten erstellt oder abgerufen sowie ausgewertet wird (insbesondere durch Hinterlegung entsprechender Daten in einer dafür vorgesehenen Datenbank z.B. auf einem Server): Geburtsdatum, Geburtszeit, Geburtsort, wahlweise auch Name und/oder Profilbild,
Erfindungsgemäß wird vorgeschlagen, dass die Datengrundlage des Persönlichkeitsprofils computergestützt/-implementiert weiter ergänzt bzw. erweitert wird, indem eine Mehrzahl von eigenständigen Charakterprofildatensätzen zum individuenspezifischen Erstellen/Generieren/Auswerten eines charakterprofildatensatzübergreifenden (insbesondere codesetübergreifenden) Persönlichkeitsprofils herangezogen und ausgewertet werden, insbesondere wenigstens fünf eigenständige Charakterprofildatensätze, insbesondere Charakterprofildatensätze betreffend die folgenden Charakterprofile: Genekey-Profil(daten), Human Design-Profil(daten), Numerologie-Profil(daten), Bazi Suan Ming-Profil(daten), Astrologie-Profil(daten). Dies begünstigt einerseits die Verarbeitung und Berücksichtigung eines hohen Grades an Komplexität und dadurch eine genauere charakterliche Kennzeichnung von einzelnen Individuen, andererseits kann auch ein gezielterer charakterlicher Abgleich unter mehreren Individuen erfolgen.

Dabei werden vorteilhaft aus der Datengrundlage eine Mehrzahl von codesetübergreifenden Persönlichkeitseigenschafts-Codes erzeugt, indem eine Mehrzahl von eigenständigen Codesets, insbesondere wenigstens fünf eigenständige Codesets genutzt werden.

Als "Persönlichkeitsprofil" ist gemäß der vorliegenden Offenbarung insbesondere ein Persönlichkeitsprofildatensatz zu verstehen, mittels welchem das jeweilige Persönlichkeitsprofil von Individuen basierend auf einer Mehrzahl von Charakterisierungs-Ansätzen beschrieben werden kann, insbesondere unter Bezugnahme auf eine Vielzahl von Codes (bzw. einen durch die entsprechenden Codes definierten Codeset) eines jeweiligen Charakterisierungs-Ansatzes, insbesondere unter Bezugnahme auf eine Vielzahl von mit einzelnen Codes korrelierten Textbausteinen (sofern eine textbasierte Beschreibung des Persönlichkeitsprofils im entsprechenden Anwendungsfall erwünscht ist). Insoweit ist hier der Begriff "Persönlichkeitsprofil" breit im Sinne von Persönlichkeit zu verstehen, und "Charakterprofil" ist eher eng im Sinne von basierend auf den jeweiligen vorbekannten Charakterisierungsansätzen ermittelter Charakterzüge eines Individuums zu verstehen.

Es ist zu verstehen, dass die Angabe des Name und/oder eines Profilbilds nicht unbedingt erforderlich ist, sondern rein optional erfolgen kann, z.B. um im Rahmen von Sichtung und Auswertung die Zuordnung der Daten zu erleichtern. Das hier beschriebene Verfahren kann ohne Angabe von Name und/oder eines Profilbild implementiert sein/werden.

Dabei beruht die Erfindung auch auf dem Konzept, einen computerimplementierbaren Ansatz bereitzustellen, mittels welchem eigenständige charakterprofildatensatzspezifische Codesets zu einem die Information des jeweiligen Charakterprofildatensatzes umfassenden Persönlichkeitsprofildatensatz, insbesondere zu charakterprofildatensatzübergreifenden Codeset kombiniert werden können, wobei vorteilhafter Weise auch ein Abgleich mit vordefinierbaren Kriterien bzw. Attributen erfolgen kann. Trotz der vergleichsweise hohen Komplexität der Auswertung des jeweiligen Persönlichkeitsprofils ermöglicht die vorliegende Erfindung auch die Bereitstellung eines Datensatzes, welcher vergleichsweise einfach/problemlos ausgelesen werden kann, insbesondere mittels gängiger Endgeräte, insbesondere ohne starke Individualisierung einer dafür vorgesehenen Software oder Hardware. Nicht zuletzt macht die Erfindung auch Ankreuztests oder (Bewerber-)Fragebögen überflüssig. Eine Personalfrage oder die Auswahl von Individuen kann insbesondere innerbetrieblich auf deutlich schlankere bzw. effizientere Weise angegangen werden.

Als "Codeset" ist gemäß der vorliegenden Offenbarung insbesondere die Menge oder Auswahl von Codes eines bestimmten vorbekannten Charakterisierungs-Ansatzes zu verstehen, also z.B. die durch den Genekeys- (Charakterisierungs-)Ansatz möglichen/nutzbaren Codes oder eine darunter getroffene Code-Auswahl. Sofern nicht anderweitig konkretisiert, betrifft der Begriff "Codeset" gemäß der vorliegenden Offenbarung die Codes nur eines der mehreren zur Auswertung herangezogenen vorbekannten (Charakterisierungs-)Ansätze. Die erfindungsgemäß erstellten oder genutzten codesetübergreifenden Persönlichkeitseigenschafts-Codes können ebenfalls als eine Code-Auswahl bezeichnet werden, jedoch überschreitet diese Code-Auswahl die durch einen jeweiligen vorbekannten Charakterisierungs-Ansatz definierten Grenzen, indem auch Codes weiterer vorbekannter Ansätze mit berücksichtigt werden. Insofern bedeutet "codesetübergreifend" gemäß der vorliegenden Offenbarung eine Code-Auswahl jedenfalls umfassend Codes mehrerer vorbekannter Charakterisierungs-Ansätze, insbesondere aller fünf hier beschriebenen vorbekannten Charakterisierungs-Ansätze.

Es ist zu verstehen, dass die erfindungsgemäße Herangehensweise bzw. Auswertung eine ganzheitliche(re) menschliche Einschätzung zu den Fähigkeiten eines Individuums ermöglicht. Bei vorbekannten Systemen z.B. wie der MBTI (Myer-Briggs)-Auswertung ist keine ungefärbte bzw. manipulationsfreie bzw. nicht-subjektive Einschätzung möglich, sondern eher nur eine Momentaufnahme momentaner Ausprägungen der Persönlichkeit. Die erfindungsgemäße Auswertung hingegen ermöglicht z.B. auch eine manipulationsfreie ganzheitliche Einschätzung des Potenzials des jeweiligen Individuums.

Dabei kann die Datengrundlage weiter ergänzt werden, indem eine Mehrzahl von eigenständigen Codesets zum individuenspezifischen Generieren/Auswählen/Auswerten von codesetübergreifenden Persönlichkeitseigenschafts-Codes genutzt werden, insbesondere wenigstens fünf eigenständige Codesets, insbesondere Codesets betreffend die folgenden Charakterprofile oder Persönlichkeitsprofildaten: Genekeys-Codeset, Human Design-Codeset, Numerologie-Codeset, Bazi Suan Ming-Codeset, Astrologie-Codeset.

Als "Charakterprofildatensatz" ist insbesondere ein durch einen der Charakterisierungsansätze gemäß dem Stand der Technik erstellbarer/erstellter Datensatz zur Beschreibung eines Charakters eines einzelnen Individuums zu verstehen. Davon grenzt sich der hier gewählte Begriff "Persönlichkeitsprofil(datensatz)" zumindest insoweit ab, als die erfindungsgemäße Beschreibung der Persönlichkeit (bzw. des Charakters) ansatzunabhängig bzw. ansatzübergreifend bzw. systemübergreifend erfolgen kann (bzw. die dafür genutzten Codes ansatzübergreifend "generiert" werden), also nicht auf nur einen einzelnen der Charakterisierungs-Ansätze gemäß dem Stand der Technik zurückgeführt werden muss, sondern aus mehreren der vorbekannten Charakterisierungs-Ansätze gebildet werden kann.

Als "(Charakterisierungs-)Ansatz" oder "(Charakterisierungs-)System" ist insbesondere jeweils eine spezifische der Herangehensweisen zur Charakterbestimmung gemäß dem Stand der Technik zu verstehen, insbesondere jene die aus dem Yijing hervorgehen, wobei diese Herangehensweisen gemäß dem Stand der Technik unabhängig voneinander angewandt werden, wobei sich die vorliegende Erfindung insbesondere im Kontext mit den folgenden Herangehensweisen bzw. Charakterisierungsansätze ausbildet: Bazi Suan Ming, Human Design, Genekeys, Numerologie, Astrologie.

Als "Kennwert" ist insbesondere ein personenbezogenes Datum zu verstehen, welches wahlweise nur durch einen Nutzer für eine momentane Verarbeitung eingegeben wird, ohne dauerhaft abgespeichert zu werden, oder welches in einer entsprechenden Datenbank zum ein- oder mehrmaligen Abrufen hinterlegt wird, beispielsweise für wunschgemäß (insbesondere mit dem jeweiligen Individuum abgestimmten) wiederholten Abgleich von Persönlichkeitsprofilen, z.B. im Rahmen eines Dauersuchauftrags für z.B. eine neue Arbeitsstelle (Jobangebot).

Als "Attribut" ist insbesondere ein fallweise anwendungsspezifisch insbesondere gemäß Vorgaben eines Auftraggebers vergebbarer Priorisierungs-/Gewichtungsparameter zu verstehen, mittels welchem eine individuelle Gewichtung von Charaktermerkmalen vorgenommen werden kann, insbesondere zum Generieren und Abgleichen eines gewichteten Persönlichkeitsprofils.

Als "Erstellen" ist hier insbesondere das Schaffen einer Datenbasis zu verstehen, z.B. in Verbindung mit dem Neuanlegen von Gruppenmitgliedern (z.B. dann, wenn ein neuer Mitarbeiter in eine größere Arbeitsumgebung mit bereits vielen weiteren Mitarbeitern integriert werden soll).

Als "Generieren" ist hier insbesondere das charakterprofildatensatzübergreifende (insbesondere codesetübergreifende) Erzeugen des Persönlichkeitsprofils zu verstehen, also das computergestützte/- implementierte Erzeugen eines Persönlichkeitsprofils, welches nicht (wie bisher) durch den jeweiligen bisher bereits vorgegebenen Codeset bzw. durch die Möglichkeiten eines jeweiligen (Charakterisierungs-)Ansatzes gemäß dem Stand der Technik limitiert ist; der Vorgang des "Generierens" kann dabei sowohl ein einzelnes Individuum als auch eine Gruppe von Individuen bzw. deren Zusammensetzung betreffen und z.B. in Abhängigkeit vordefinierbarer Kriterien/Attribute zu anderen Ergebnissen bzw. anderen Gruppenzusammensetzungen führen.

Als "Abgleichen" ist dabei insbesondere ein Vergleichen von übereinstimmenden Persönlichkeitseigenschafts-Codes von wenigstens zwei Individuen und eine Einordnung des jeweiligen Individuums hinsichtlich dessen Grad der Übereinstimmung oder Kompatibilität (bzw. charakterlicher Ergänzung) mit den Persönlichkeitseigenschaften des wenigstens einen weiteren Individuums zu verstehen. Dabei kann dem jeweiligen Individuum auch eine Kompatibilitätskennzahl zugewiesen werden, insbesondere spezifisch in Hinblick auf eine Gruppe ausgewählter Individuen oder eine entsprechende durch die auszuwählenden Individuen zu bildende Gruppe; über eine solche Kompatibilitätskennzahl kann dabei auch ein potentieller Konflikt unter einzelnen Mitgliedern einer Gruppe abgebildet bzw. im Sinne einer Risikoabschätzung antizipiert werden.

Die vorliegende Erfindung kann auch im Rahmen eines individualisierten Abgleichens von Charakterprofilen implementiert sein, also dadurch zur Anwendung kommen, dass eine bereits gemäß den hier beschriebenen Merkmalen erstellte Datenbasis eines Individuums mit den Persönlichkeitsprofilen weiterer Individuen abgeglichen wird, insbesondere in Abhängigkeit vordefinierbarer Kriterien. Anders ausgedrückt: Das Erstellen eines Persönlichkeitsprofils als Vorgang als solcher ist nicht notwendigerweise Bestandteil eines erfindungsgemäßen Verfahrens, sobald auf einen bereits erstellten Datensatz zurückgegriffen wird, z.B. dann, wenn eine Zusammenstellung von Gruppen (z.B. Mitarbeiter-Teams) erneut durch Maßnahmen wie z.B. Job-Rotation oder dergleichen vorgenommen wird, z.B. innerhalb einer Organisation, in welcher für die einzelnen Individuen bereits ein Persönlichkeitsprofildatensatz gemäß der vorliegenden Offenbarung generiert wurde.

Als "Profiling" ist hier insbesondere ein Oberbegriff für alle Maßnahmen zur Ermittlung des Persönlichkeitsprofils zu verstehen. Einzelne Charaktereigenschaften können auch als Profiling-Attribute bezeichnet oder verstanden werden.

Personifizierte Begriffe, soweit sie hier nicht im Neutrum formuliert sind, können im Rahmen der vorliegenden Offenbarung alle Geschlechter betreffen. Etwaige hier verwendete englischsprachige Ausdrücke oder Abkürzungen sind jeweils branchenübliche Fachausdrücke und sind dem Fachmann in englischer Sprache geläufig. Etwaige dazu synonym verwendete/verwendbare deutschsprachige Begriffe können hier der Vollständigkeit halber in (Klammern) angegeben werden, oder vice versa, beispielsweise bezüglich des Begriffs Profiling (Persönlichkeitsprofilbestimmung insbesondere unter Anwendung von Attributen bzw. Gewichtungsfaktoren), Matching (Abgleichen) oder Scoring (Grad, Überlappung, Reihenfolge bzw. Rangliste).

Gemäß dem Stand der Technik kann ein Charakterprofil (Summe aller verfügbaren bzw. bestimmbaren Charaktermerkmale eines Individuums) durch jeweils einen (einzigen) der folgenden Datensätze oder Ansätze definiert werden: Bazi Suan Ming-Ansatz, Human Design-Ansatz, Genekeys-Ansatz, Numerologie-Ansatz, Astrologie-Ansatz. Jeder dieser Ansätze kann dabei auf einem spezifischen Datensatz oder auch Codeset beruhen, wobei der/das jeweilige Codeset vordefinierte/vordefinierbare Codes mit bestimmten Charaktermerkmalen korreliert. Davon ausgehend ermöglicht die vorliegende Erfindung, diese Ansätze miteinander zu kombinieren, insbesondere um eine breitere Datengrundlage effizient/effektiv nutzen zu können, für eine noch treffendere Persönlichkeitsprofilbestimmung bzw. für einen noch gezielteren Abgleich von Charaktereigenschaften mehrerer Individuen untereinander. Insbesondere können alle diese fünf Ansätze derart miteinander kombiniert werden (insbesondere indem fünf komplette eigenständige Codesets genutzt bzw. ausgewertet werden), dass daraus ein erweiterter Persönlichkeitsprofildatensatz generiert werden kann, wobei dieser erweiterte Persönlichkeitsprofildatensatz vorteilhaft durch eine Vielzahl von codesetübergreifenden bzw. ansatzunabhängigen Codes beschrieben wird (also Codes, die losgelöst von den hier beschriebenen fünf standardmäßigen Ansätzen sind/werden), und wobei die jeweiligen individuenspezifischen Codes dieses erweiterten Persönlichkeitsprofildatensatzes gemäß einer optionalen Implementierung vorteilhaft mit individuell gewichtbaren (Charakter-)Kriterien im Sinne eines Matching und Scoring mehrerer Individuen in der Art einer Priorisierung abgeglichen werden können, zum Bestimmen von wenigstens zwei miteinander harmonierenden oder sich ergänzenden Individuen in Abhängigkeit von vordefinierbaren Kriterien. Im Folgenden werden fünf Ansätze gemäß dem Stand der Technik vorgestellt, basierend auf welchen die erfindungsgemäße Datengrundlage generiert und ausgewertet werden kann:

Der so genannte Human Design-Ansatz (persönlichkeitsdiagnostisches System) beruht dabei auf einer Methode zur Charakterprofilerkennung, welche Ansätze aus westlicher Astrologie, aus indischbrahmanischer Chakren-Lehre, aus der Kabbala (eine überlieferte Schrift aus dem Judentum) und aus dem Yijing (bzw. I'Ging, der vermutlich älteste überlieferte chinesische Text) vereint. Dazu werden die Geburtsdaten einer Person genutzt (insbesondere Geburtsjahr, -monat, -tag, -stunde sowie -ort). Es werden 64 Codes (bzw. Hexagramme) genutzt, welche in Verbindung mit Sternzeichen und in Verbindung mit sechs Variablen (bzw. Facetten) mit je zwei Aspekten, 12 Profilen, fünf Typen, fünf Strategien und 768 Inkamationskreuzen eine totale Kombination von ca. 2 Milliarden Aspekten bzw. Merkmalen ergibt; aus dieser Vielzahl von Aspekten kann ein individuenspezifisches Charakterprofil ermittelt werden. Vorteilhaft werden gemäß der vorliegenden Erfindung aus diesem Ansatz bzw. System alle Codes entnommen/generiert bzw. genutzt. Es hat sich im Rahmen der vorliegenden Erfindung gezeigt, dass der Human Design-Ansatz bzw. darauf basierend generierte Codes vorteilhaft vornehmlich hinsichtlich einer Auswertung einer/der mentalen Charakterebene genutzt bzw. implementiert werden kann.

Der so genannte Bazi Suan Ming-Ansatz beruht dabei auf einer Methode der Charakterbestimmung, bei welcher das Verhalten, die familiären und sozialen Beziehungen, die Gesundheit, Potenziale sowie zukünftige Entscheidungen im Vordergrund stehen. Dazu werden die Geburtsdaten einer Person, wie das Geburtsjahr, der Geburtsmonat, -tag, -stunde sowie -ort verwendet. Diese Daten werden in vier Paare übersetzt; jedes Paar nennt sich Säule mit zwei Charakteren. Es werden zehn Profile (fünf Element + zwei Variablen), 12 Tierkreiszeichen und vier Säulen mit je 60 möglichen Kombinationen zu Grunde gelegt (dies führt zu wenigstens ca. 30.000 Möglichkeiten für unterschiedliche Charaktermerkmale). Aus all diesen Aspekten kann ein Charakterprofil ermittelt werden. Vorteilhaft werden aus diesem Ansatz bzw. System zehn (10) Codes (Teilauswahl) gemäß der vorliegenden Erfindung entnommen/generiert bzw. genutzt. Es hat sich im Rahmen der vorliegenden Erfindung gezeigt, dass der Bazi Suan Ming-Ansatz bzw. darauf basierend generierte Codes vorteilhaft vornehmlich hinsichtlich einer Auswertung einer/der transzendenten Charakterebene genutzt bzw. implementiert werden kann. Auch der Bazi Suan Ming-Ansatz kann auf die 64 Hexagramme des Yijing zurückgeführt werden.

Der so genannte Astrologie-Ansatz beruht dabei auf einer Methode der Charakterbestimmung, bei welcher das Bewusstsein und das Verhalten beeinflussende energetische Einflüsse sowie Wechselwirkungen mit Innen- und Außenwelt, sowie Talentmerkmale und Ziele im Leben im Vordergrund stehen. Dazu werden die Geburtsdaten einer Person, wie das Geburtsjahr, der Geburtsmonat, -tag, -stunde sowie -ort verwendet. Die Methode basiert auf 12 Planeten, 12 Häuser, zehn Tierkreiszeichen, den drei Energien, vier Achsen, sechs Aspekten und den vier Elementen. Daraus ergeben sich wenigstens 1 Millionen mögliche Kombinationen. Aus allen Aspekten kann das jeweilige Charakterprofil bestimmt werden. Vorteilhaft werden aus diesem Ansatz bzw. System wenigstens zehn (10) oder 20 Codes (Teilauswahl) gemäß der vorliegenden Erfindung entnommen/generiert bzw. genutzt, wahlweise alle verfügbaren Codes. Es hat sich im Rahmen der vorliegenden Erfindung gezeigt, dass der Astrologie-Ansatz bzw. darauf basierend generierte Codes vorteilhaft vornehmlich hinsichtlich einer Auswertung eines Zusammenhangs der einzelnen Charakterebenen genutzt bzw. implementiert werden kann, insbesondere für eine Verknüpfung bzw. Korrelation der einzelnen Charakterebenen sorgen kann. Demnach kann es besonders vorteilhaft sein, die aus diesem Ansatz genutzten Codes am Ende des hier beschriebenen Verfahrens auszuwerten bzw. mit den aus den anderen Ansätzen entnommenen Codes zu korrelieren. Auch der Astrologie-Ansatz kann auf die 64 Hexagramme des Yijing zurückgeführt werden.

Der so genannte Genekeys-Ansatz beruht dabei auf einer Methode, welche am Human Design-Ansatz angelehnt ist, basierend auf der Übereinstimmung bzw. Korrelation einer/der DNA mit den im Yijing beschriebenen 64 Hexagrammen bzw. daraus generierter Codes und der wechselseitigen Wirkungsweise eines umgebenden, kollektiven Resonanzfeldes und eigenen Befindlichkeiten bzw. Handlungsweisen gemäß unbewussten, archaischen Verhaltensmustern. Die Methode basiert auf den 64 Hexagrammen bzw. Codes mit sechs Variablen und fünf Aspekten und 13 verschiedenen Positionen mit vier Aspekten. Hieraus ergeben sich wenigstens 1.000.000 mögliche Kombinationen. Vorteilhaft werden aus diesem Ansatz bzw. System dreizehn (13) Codes (Teilauswahl) entnommen/generiert bzw. genutzt. Es hat sich im Rahmen der vorliegenden Erfindung gezeigt, dass der Genekeys-Ansatz bzw. darauf basierend generierte Codes, insbesondere dreizehn (13) Codes, vorteilhaft vornehmlich hinsichtlich einer Auswertung einer/der emotionalen Charakterebene genutzt bzw. implementiert werden kann/können. Es ist zu verstehen, dass diese 13 Codes auch noch mit einer Variabilität (insbesondere sechs unterschiedliche Zustände) multipliziert werden können.

Der so genannte Numerologie-Ansatz beruht dabei auf einer Methode bzw. einem Zahlensystem, bei welcher einzelnen Zahlen oder Zahlenkombinationen bestimmte Bedeutungen bzw. symbolische Funktion zugewiesen werden, und welche/s beliebig häufige Kombinationen von Zahlen bzw. entsprechender Korrelationen ermöglicht, so dass unendlich viele Kombinationen möglich sind (theoretisch). Es hat sich im Rahmen der vorliegenden Erfindung gezeigt, dass vorteilhaft (lediglich) zwei spezifische Systeme (insbesondere jene nach Linda Goodman und Daniel Hassler) aus der Vielzahl von Systemen genutzt/herangezogen werden mögen, die jeweils maßgeblich die Persönlichkeit charakterisieren können (insbesondere jene betreffend Zusammenhänge zwischen Geburtszahlen, Charaktermerkmalen, Verhaltensweisen, Krankheiten und Aspekten des Lebens und Zusammenlebens). Diese Methoden nutzen jeweils Zahlen von 1 bis 10, bzw. es werden die Geburtsdaten in mathematischem Sinne verwendet (Auszählen der Zahlen von 0-9 und Zuordnung zu einem System mit 10 Eigenschaftsattributen). Diese Informationen können im Sinne einer Zusatzinformation mit in die hier beschriebene Matrix einfließen und das Charakterprofil ergänzen. Vorteilhaft werden zwanzig (20) Codes (Teilauswahl) aus diesem Ansatz bzw. System gemäß der vorliegenden Erfindung genutzt/generiert (zehn erste Codes aus der ersten Methode, insbesondere jener nach Linda Goodman, und zehn zweite Codes aus der zweiten Methode, insbesondere jener nach Daniel Hasler), insbesondere um diese in Verbindung mit den ausgewählten Codes der weiteren Ansätze zu nutzen, insbesondere um diese in eine/die Matrix zu integrieren. Es hat sich im Rahmen der vorliegenden Erfindung gezeigt, dass der Numerologie-Ansatz bzw. darauf basierend generierte Codes vorteilhaft vornehmlich hinsichtlich einer Auswertung einer/der körperlichen Charakterebene genutzt bzw. implementiert werden kann. Es ist zu verstehen, dass der Numerologie-Ansatz nicht auf die 64 Hexagramme des Yijing zurückgeführt wird.

Erwähnenswert ist, dass das oben bereits erwähnte Yijing (I'Ging), das so genannte Buch der Wandlungen, ein wohl über 4.000 Jahre alter klassischer chinesischer Text mit einer Bezugnahme auf Charakterprofile, die Basis für mehrere der hier beschriebenen fünf vorbekannten Ansätze liefert, je nach Auslegung der Überlieferung auch als Basis für alle fünf hier beschriebenen vorbekannten Ansätze erachtet werden kann; das Yijing bzw. dessen ältester Bestandteil liefert dabei mit 64 Hexagrammen (aus sechs durchgezogenen oder gestrichelten Linien zusammengesetzte Zeichen) eine entsprechende Anzahl von Bildern oder Grundzeichen oder Gruppen, welche als Charakterzüge oder auch Handlungsempfehlungen interpretiert werden können und in ihrer Systematik durch Zusatzhinweise erweitert werden können und dadurch bereits 384 Lebens- oder Charaktersituationen beschreiben können, und welche dabei auch durch Wandel einzelner Linien in die anderen Bilder bzw. Grundzeichen durchübergehen können, was zu einer Anzahl von wenigstens 4.096 Variationen führt. Diese Komplexität ermöglicht eine Basis, die für mehrere oder sogar alle der hier beschriebenen fünf vorbekannten Ansätze herangezogen wird bzw. werden kann. Die vorliegende Erfindung basiert auf diesen Ansätzen auf, indem die individuenspezifischen Ergebnisse eines jeweiligen Ansatzes miteinander korreliert und verrechnet werden, zum Generieren eines charakterprofildatensatzübergreifenden Persönlichkeitsprofils, welches eine noch gezieltere Charakterbestimmung und davon ausgehend auch das hier beschriebene möglichst gezielte Abgleichen von Charaktermerkmalen insbesondere im Zusammenhang mit dem Bilden von harmonisierenden Teams oder Gruppen aus zahlreichen Individuen ermöglicht. Auf diese Weise können nicht zuletzt auch sehr effiziente/effektive Teams für sehr komplexe Aufgaben oder Fragestellungen computergestützt "zusammengewürfelt" werden.

Es ist zu verstehen, dass sichergestellt werden kann, dass jeder dieser fünf vorbekannten Einzelansätze ausreichende Informationen liefert, um die hier beschriebenen erfindungsgemäßen Codes zu generieren und auszuwählen, so dass der hier beschriebene Persönlichkeitsprofildatensatz aus fünf Einzelansatzcharakterprofilen generiert werden kann, die jeweils aus einem der hier beschriebenen fünf vorbekannten Ansätze hervorgehen. Dabei kann auch sichergestellt werden, dass eine potentielle Redundanz von Codes oder Charaktermerkmalen aus den fünf Einzelansätzen vermieden wird, und dass keine sich widersprechenden (oder nicht miteinander kompatiblen) Codes aus den Einzelansätzen ermittelt werden, insbesondere indem die Daten bzw. Codes in einer mehrdimensionalen Matrix verwaltet werden, bei welcher der jeweilige Persönlichkeitsprofildatensatz auf wenigstens drei Ebenen angelegt wird bzw. daraus zusammengesetzt wird: Ebene des Körpers bzw. körperliche Ebene (erste Ebene einschließlich Unterebenen), Ebene des Verstandes und der Persönlichkeit (zweite Ebene einschließlich Unterebenen) und Ebene des Geistes und der Seele (dritte Ebene einschließlich Unterebenen). Die hier beschriebenen vorbekannten Einzelansätze des Human Designs, der Genekeys und der Astrologie, sowie des Bazi Suan Ming und der Numerologie können ebenfalls in mehreren Ebenen unterscheiden, insbesondere in diesen drei hier genannten Ebenen. Auf diese Weise kann unabhängig davon, ob die Analyse durch einen jeweiligen Einzelansatz oder durch die hier beschriebene Gesamtschau erfolgt, eindeutig zugeordnet werden, welche Eigenschaft auf welcher Ebene zu berücksichtigen ist. Dabei hat es sich im Rahmen der vorliegenden Erfindung als zielführend erwiesen, etwaige Redundanzen dann zu vereinigen, wenn sie auf der gleichen Ebene vorliegen; etwaige Widersprüche auf einer Ebene können auch dadurch ausgeschlossen werden, dass die herangezogenen Einzelansätze auf einen gemeinsamen Ursprung zurückgeführt werden können. Falls dennoch ein Widerspruch von Eigenschaften oder Codes aufkommen sollte, kann am Einzelfall eine Plausibilitätsprüfung erfolgen, insbesondere durch Anwendung eines Mehrheitsprinzips.

Vorteilhaft ist eine/die hier beschriebene Matrix (erfindungsgemäß generierter Datensatz, generierte Datensatzstruktur) mehrdimensional. Vorteilhaft umfasst der hier beschriebene Datensatz auch wenigstens eine über eine Übersetzung von Codes in Textbausteine hinausgehende Dimension, insbesondere basierend auf den Daten/Informationen, welche durch Vereinheitlichung und Kombination der hier beschriebenen fünf Ansätze/Systeme generiert werden können und dann abgeglichen werden können. Vorteilhaft können tendenziell als eher unvereinbar erachtete Charaktereigenschaften wie z.B. schüchtern einerseits und extrovertiert andererseits koexistieren, insbesondere auf unterschiedlichen Ebenen des ermittelten Persönlichkeitsprofils. Insofern bedeutet hier "mehrdimensional" auch, dass der generierte Datensatz ermöglicht, eine Vielzahl von Ebenen eines Persönlichkeitsprofils zu beachten bzw. zu generieren, insbesondere ausgehend von den hier explizit beschriebenen vier Ebenen (emotional, mental, körperlich, transzendent) zuzüglich der Astrologie-Betrachtung wenigstens dreizehn (13) Ebenen.

Es ist zu verstehen, dass die vorliegende Erfindung einerseits auf dem Konzept beruht, vorbekannte Ansätze vorrangig jeweils hinsichtlich einer der folgenden vier Ebenen auszuwerten: emotionale, mentale, körperliche und transzendente Ebene. Gleichwohl gibt es unter diesen Ebenen Abhängigkeiten, was z.B. an folgendem Bild verdeutlicht werden kann: eine schüchterne Person kann gleichwohl ein gefährliches abschreckendes Aussehen haben.

Ein Nutzer der erfindungsgemäßen Verfahren und Vorrichtungen bzw. Systemkomponenten kann die Geburtsdaten (insbesondere Tag, Zeit und Ort) und einen/den Namen über eine Benutzerschnittstelle eingeben, insbesondere über die hier beschriebene Benutzerschnittstelle. Danach erfolgt eine Übersetzung bzw. Transformation der hier beschriebenen ausgewählten Codes der fünf einzelnen vorbekannten Systeme/Ansätze, bevorzugt in Textbausteine, so dass ein einerseits codebasiertes und andererseits auch (aus-)lesbares individuenspezifisches Persönlichkeitsprofil erstellt wird, welches für einen optimierten Abgleich genutzt werden kann. In einem optionalen weiteren Schritt des Abgleichens kann durch Scoring und Matching mit weiteren individuell vordefinierbaren Kriterien das charakterspezifische Zuordnen von Individuen zu Paaren oder Gruppen weiter verfeinert bzw. optimiert oder für bestimmte Themen und Aufgabengebiete angepasst werden.

Der hier beschriebene generierter Datensatz (bzw. die generierte Datensatzstruktur, oder, mit anderen Worten: die so genannte Matrix) kann durch standardmäßig verwendete Endgeräte genutzt bzw. verarbeitet werden. Eine Datenpflege und -erweiterung kann individuenspezifisch oder zentral vorgenommen werden. Beispielsweise werden als hinterlegte/abgespeicherte Daten die von den jeweiligen Nutzern eingegebenen Daten abgespeichert, sowie die darauf basierend ermittelten Charakterprofile, jedoch ohne Matching und Scoring-Maßnahme, wahlweise auch ein Profilbild, welches jedoch nicht notwendigerweise in der Datensatzstruktur der Persönlichkeitsprofile abgelegt sein muss.

Die vorliegende Erfindung ermöglicht auch eine Individualisierung der Abgleichfunktion auf einfache Weise, insbesondere indem für den Abgleich der Persönlichkeitsprofile wenigstens ein Attribut (Priorisierungs-/Gewichtungsparameter) vorgegeben wird. Dabei kann eine "Übersetzung" von den vom jeweiligen Nutzer oder Auftraggeber (Persönlichkeitsprofile suchende Institution oder Person) gewünschten Attributen hin zur Gewichtung einzelner Codes/Eigenschaften erfolgen, insbesondere indem vom jeweiligen Nutzer oder Auftraggeber vorgegebenen die (Profiling-)Attribute mit dem erstellten Persönlichkeitsprofil abgeglichen werden und Matches bzw. Übereinstimmungen ermittelt werden (diese Systematik des Matching wird bevorzugt einheitlich bzw. unverändert angewandt). Je mehr Attribute vom Nutzer/Auftraggeber vorgegebenen werden, desto spezifischer kann die Suche nach dem geeigneten Individuum ausgestaltet werden. Diese Maßnahme kann dabei in der Systematik unabhängig von spezifisch ausgewählten Attributen bleiben, d.h., der Prozess zur Persönlichkeitsprofilbestimmung und der Output werden nicht beeinflusst (nicht notwendigerweise).

Dabei kann auch eine Charakterisierung einer Gruppenzusammensetzung erfolgen, um darauf basierend ein besonders geeignetes Individuum einer Gruppe zuordnen zu können (Matching nicht nur von zwei einzelnen Individuen, sondern Auswahl eines Individuums, das besonders gut zu einer Gruppe von z.B. bereits ca. zehn Personen passt). Dazu wird bevorzugt zunächst ein Scoring von der bereits bestehenden Gruppe erstellt, gemäß den vom Nutzer/Auftraggeber vorgegebenen Attributen. Dann wird ein Scoring des abzugleichenden Individuums zu diesen Attributen erstellt und mit dem Scoring der bestehenden Gruppe verglichen. Davon ausgehend wird das beste Match ausgewählt, bzw. es werden diejenigen Individuen ausgewählt, bei welchen die meisten Übereinstimmungen insbesondere mit den Attributvorgaben ermittelt werden können. Diese Maßnahme kann unabhängig von der Anzahl der Individuen der Gruppe auf dieselbe Art und Weise angewandt werden. Diese Maßnahme kann wahlweise zur Ergänzung einer Gruppe durch wenigstens ein Individuum und/oder auch zur Bildung einer neuen Zusammensetzung einer Gruppe (Austausch wenigstens eines Individuums) angewandt werden.

Dabei kann die Datengrundlage weiter ergänzt werden, indem eine Mehrzahl von Persönlichkeitseigenschafts-Codes erzeugt/ermittelt und individualisiert mit dem jeweiligen Individuum korreliert werden, wobei die Persönlichkeitseigenschafts-Codes unter anderem unter Bezugnahme auf die astrologischen Koordinaten erzeugt/ermittelt werden und jeweils durch wenigstens ein Merkmal aus der folgenden Gruppe charakterisiert und voneinander unterschieden/abgegrenzt werden: Variable, Modalität, Archetyp, Hauptzustand, Nebenzustand, Identifizierungs-/Charaktermerkmal; wobei der Abgleich der Persönlichkeitsprofile von wenigstens zwei Individuen unter Bezugnahme sowohl auf zumindest eine Teilauswahl der ermittelten Persönlichkeitseigenschafts-Codes des jeweiligen Individuums als auch unter Bezugnahme auf charakterprofildatensatzspezifische Charakter-Codes erfolgt. Es hat sich gezeigt, dass es zielführend ist, aus den einzelnen vorbekannten Ansätzen, zumindest jeweils aus dem Astrologie- und dem Human Design- und dem Bazi Suan Ming- Ansatz, eine Codeauswahl zu generieren, und diese Codeauswahl mit Codes des Numerologie-Ansatzes und Codes des Genekeys-Ansatzes zu kombinieren, insbesondere zum Generieren von 64 Persönlichkeitseigenschafts-Codes. Der erfindungsgemäße Ansatz ermöglicht dabei auch, ausgehend von jeweils 64 Hexagrammen oder daraus generierter Codes von vier vorbekannten Ansätzen und ausgehend von Codes des Numerologie-Ansatzes (insbesondere ausgehend von ersten und zweiten Codes des Numerologie-Ansatzes) aussagekräftigere 64 Persönlichkeitseigenschafts-Codes zu generieren, ohne dass dabei ein Widerspruch oder Konflikt der einzelnen Codes untereinander auftritt. Dabei kann der Astrologie-Ansatz auch als ein die vier Basisebenen (emotionale, mentale, körperliche, transzendente Ebene) übergreifender Ansatz beschrieben werden, in der Art einer ansatzverknüpfenden fünften Ebene.

Insofern beruht die vorliegende Erfindung auch auf dem Konzept, ausgehend von einer Mehrzahl von Ansätzen, die jeweils auf den 64 Hexagrammen des Yijing basieren, eine Codeauswahl zu treffen, diese ausgewählten Codes auch mit Codes des Numerologieansatzes zu kombinieren, und daraus 64 Persönlichkeitseigenschafts-Codes zu generieren, wobei die in diesen 64 Persönlichkeitseigenschafts-Codes enthaltene Information vorteilhaft auf neun (9) oberbegriffsartige Hauptcodes zusammengefasst werden kann, wobei vorteilhaft wahlweise nur die neun (9) oberbegriffsartigen Hauptcodes zur Ausgabe/Beschreibung eines Persönlichkeitsprofils genutzt bzw. ausgegeben werden (bzw. entsprechende Textbausteine z.B. zusammengefasst auf einer halben DinA4-Seite), insbesondere um die Informationsverarbeitung und -nutzung z.B. durch im Personalmanagement oder Recruiting tätige Personen zu erleichtern. Die neun (9) oberbegriffsartigen Hauptcodes können dabei vorteilhaft in einer 3x3-Martix dargestellt werden, insbesondere bei farblicher Hervorhebung, insbesondere mit einer/der Darstellung der mentalen Ebene oberhalb von einer/der Darstellung der emotionalen Ebene oberhalb von einer/der Darstellung der körperlichen Ebene, insbesondere mit einer/der Darstellung der transzendenten Ebene im vertikal-mittigen Bereich.

Hauptcodes können dabei z.B. auch in Abhängigkeit von der Relevanz für die jeweilige Zielgruppe definiert sein/werden, beispielsweise indem die Hauptcodes suchanfragenspezifisch ausgewählt bzw. vorgeschlagen werden, insbesondere computergestützt. Vorteilhaft werden zwei Hauptcodes aus einem jeweiligen Bereich bzw. einer jeweiligen der vier hier explizit beschriebenen Ebenen definiert (emotional, mental, körperlich, transzendent), zuzüglich eines Codes aus dem Astrologie-Ansatz. Dies liefert nicht zuletzt auch eine breite Anwendungsmöglichkeit basierend auf den gleichen Hauptcodes.

Dabei kann das jeweilige Persönlichkeitsprofil durch Persönlichkeitseigenschafts-Codes in einem Persönlichkeitsprofildatensatz in Form von wenigstens zwei Arten von Codes individualisiert hinterlegt sein/werden, nämlich über erste Codes und zweite Codes, wobei der Abgleich des Persönlichkeitsprofils unter Bezugnahme sowohl auf zumindest eine Teilauswahl der ersten als auch auf zumindest eine Teilauswahl der zweiten Codes erfolgen kann.

Dabei können die Persönlichkeitseigenschafts-Codes derart in einem/dem Persönlichkeitsprofildatensatz insbesondere in Matrix-Form hinterlegt sein/werden, dass das (jeweilige) Persönlichkeitsprofil im Persönlichkeitsprofildatensatz individualisiert über die Codes abgebildet wird (abbildbar ist), wobei mittels des Persönlichkeitsprofildatensatzes eine Mehrzahl von Persönlichkeitseigenschaften mit dem jeweiligen Individuum basierend auf wenigstens zwei Arten von Persönlichkeitseigenschafts-Codes derart korreliert werden, dass das jeweilige individuenspezifische Persönlichkeitsprofil durch die folgenden codespezifisch korrelierten Identifizierungs-/Charaktermerkmale weiter individualisiert/individualisierbar ist: erste Codes betreffend erste Identifizierungsmerkmale und zweite Codes betreffend zweite Identifizierungsmerkmale; wobei der Abgleich der Persönlichkeitsprofile von wenigstens zwei Individuen unter Bezugnahme sowohl auf zumindest eine Teilauswahl der ersten als auch auf zumindest eine Teilauswahl der zweiten Codes des jeweiligen Individuums erfolgt. Vorteilhaft wird eine Teilauswahl von ersten und zweiten Codes aus dem Numerologie-Ansatz vorgenommen, insbesondere erste Codes zurückgeführt auf Goodman und zweite Codes zurückgeführt auf Hasler.

Die vorliegende Erfindung beruht auch auf dem Konzept, eine Datenbank zu schaffen, in welcher für das jeweilige Individuum eine ansatzspezifische Codeauswahl derart getroffen wird, dass wenigstens die hier beschriebenen fünf Ansätze miteinander kombiniert werden können und im Ergebnis eine Ausgabe von 64 Codes erfolgen kann. Insofern umfasst die Datenbank individuenspezifisch abgelegte erste Codes aus dem Human Design-Ansatz, zweite Codes aus dem Genekeys-Ansatz, dritte Codes aus dem Bazi Suan Ming-Ansatz, vierte Codes aus dem Numerologie-Ansatz, und fünfte Codes aus dem Astrologie-Ansatz (Reihenfolge der Aufzählung hier nur beispielhaft), als heranzuziehende auszuwertende Daten, und daraus werden die hier codesetübergreifenden Codes generiert, insbesondere 64 Codes, von denen optional lediglich neun (9) Hauptcodes zur leichteren/kürzeren überblickartigen Persönlichkeitsprofilbeschreibung genutzt werden können, und für die komplette Persönlichkeitsprofilbeschreibung werden bevorzugt alle 64 Codes genutzt.

Dabei können aus der Datengrundlage (bzw. aus den astrologischen Koordinaten und/oder wenigstens einem astrologischen Parameter) die Mehrzahl von Persönlichkeitseigenschafts-Codes erzeugt/ermittelt werden, die für das jeweilige Individuum individualisiert vergeben werden und derart in einem Persönlichkeitsprofildatensatz insbesondere in Matrix-Form hinterlegt sind/werden, dass das (jeweilige) Persönlichkeitsprofil in der Matrix individualisiert über die Codes abgebildet wird (abbildbar ist), wobei mittels der Matrix eine Mehrzahl von Persönlichkeitseigenschaften mit dem jeweiligen Individuum basierend auf wenigstens zwei Arten von Persönlichkeitseigenschafts-Codes derart korreliert werden, dass das Persönlichkeitsprofil durch die folgenden codespezifisch korrelierten Identifizierungs-/Charaktermerkmale weiter individualisiert/individualisierbar ist: erste Identifizierungsmerkmale definiert durch erste Codes basierend auf Variablen und Modalitäten, zweite Identifizierungsmerkmale definiert durch zweite Codes basierend auf Archetypen und Hauptzuständen und Nebenzuständen; wobei der Abgleich der Persönlichkeitsprofile von wenigstens zwei Individuen unter Bezugnahme sowohl auf zumindest eine Teilauswahl der ersten als auch auf zumindest eine Teilauswahl der zweiten Codes des jeweiligen Individuums erfolgt.

Gemäß einer Ausführungsform basiert wenigstens einer der eigenständigen Charakterprofildatensätze auf dem Yijing und stellt eine Mehrzahl von Codes basierend auf den 64 Hexagrammen des Yijing bereit, aus welchen eine Codeauswahl für ein Codeset getroffen wird (insbesondere für einen bezüglich einer der folgenden drei Charakterebenen ausgewählten Codeset: emotionale, mentale oder transzendente Charakterebene), welcher den entsprechenden eigenständigen Charakterprofildatensatz charakterisiert und welcher eingerichtet ist, mit wenigstens einem Codeset eines der weiteren eigenständigen Charakterprofildatensätze korreliert zu werden. Dies begünstigt nicht zuletzt eine vorteilhafte Herangehensweise für eine kompatible Nutzung der in den einzelnen Ansätzen enthaltenen Informationen.

Gemäß einer Ausführungsform basieren wenigstens vier der eigenständigen Charakterprofildatensätze auf dem Yijing und stellen eine Mehrzahl von Codes jeweils basierend auf den 64 Hexagrammen des Yijing bereit, wobei diese Codes mit Codes des Numerologie-Ansatzes bzw. der Numerologie-Profildaten kombiniert werden, zum Bilden der codesetübergreifenden Persönlichkeitseigenschafts-Codes. Dies ermöglicht auch eine Verknüpfung von Yijing-basierten Ansätzen mit Ansätzen, die nicht auf dem Yijing basieren.

Gemäß einer Ausführungsform basieren vier der eigenständigen Charakterprofildatensätze auf dem Yijing, wobei aus jeweils einem dieser Charakterprofildatensätze Codes betreffend eine der folgenden Charakterebenen entnommen oder ausgewählt werden: transzendente Charakterebene, emotionale Charakterebene, körperliche Charakterebene, mentalen Charakterebene. Dies erleichtert nicht zuletzt eine Entnahme der aus den einzelnen Ansätzen hervorgehenden Informationen auf sehr umfassende Weise.

Gemäß einer Ausführungsform basieren vier der eigenständigen Charakterprofildatensätze auf dem Yijing, wobei ein erster Codeset aus dem Human Design-Ansatz und ein zweiter Codeset aus dem Genekeys-Ansatz und ein dritter Codeset aus dem Bazi Suan Ming-Ansatz generiert oder verwendet werden, zum Bilden eines Codeset, welcher mit einem vierten Codeset aus dem Astrologie-Ansatz korreliert wird, und wobei ein weiterer Codeset aus dem Numerologie-Ansatz generiert oder verwendet wird, zum Bilden der codesetübergreifenden Persönlichkeitseigenschafts-Codes. Diese Systematik ermöglicht nicht zuletzt auch eine besonders zielführende Kombination bzw. Zusammenführung der Ansätze zum erfindungsgemäß generierten Persönlichkeitsprofildatensatz.

Gemäß einer Ausführungsform basieren vier der eigenständigen Charakterprofildatensätze auf dem Yijing, wobei ein erster Charakterprofildatensatz auf dem Human Design-Ansatz basiert und einen ersten Codeset liefert, welcher eine mentale Charakterebene beschreibt, wobei ein zweiter Charakterprofildatensatz auf dem Genekeys-Ansatz basiert und einen zweiten Codeset liefert, welcher eine emotionale Charakterebene beschreibt, wobei ein dritter Charakterprofildatensatz auf dem Bazi Suan Ming-Ansatz basiert und einen dritten Codeset liefert, welcher eine transzendente Charakterebene beschreibt, wobei ein vierter Charakterprofildatensatz auf dem Numerologie-Ansatz basiert und einen vierten Codeset liefert, welcher eine körperliche Charakterebene beschreibt. Diese Gewichtung ermöglicht nicht zuletzt auch eine umfassende Auswertung der in den einzelnen Ansätzen sichtbar werdenden Informationen, insbesondere bei guter Kompatibilität miteinander, um diese dann auch in Kombination mit dem Astrologie-Ansatz zum erfindungsgemäß generierten Persönlichkeitsprofildatensatz konsolidieren zu können.

Gemäß einer Ausführungsform werden zunächst drei eigenständige Charakterprofildatensätze, die jeweils auf dem Yijing basieren, miteinander kombiniert, zum Bilden von ersten codesetübergreifenden Codes, und daraufhin werden ein/der auf dem Numerologie-Ansatz basierende Charakterprofildatensatz und ein/der auf dem Astrologie-Ansatz basierende Charakterprofildatensatz kombiniert, zum Bilden von zweiten codesetübergreifenden Codes, wobei das Persönlichkeitsprofil aus den ersten und zweiten codesetübergreifenden Codes generiert wird. Diese Vorgehensweise ermöglicht auch eine zielführende Integration des Numerologie-Ansatzes in die weiteren Yijing-basierten Ansätze.

Gemäß einer Ausführungsform basieren vier der eigenständigen Charakterprofildatensätze auf dem Yijing und liefern jeweils ein Codeset, wobei diese vier Codesets zu einem korrelierten Codeset zusammengeführt werden, wobei ein weiterer eigenständiger Charakterprofildatensatz auf einem/dem Numerologie-Ansatz basiert und einen weiteren Codeset liefert, welcher mit dem korrelierten Codeset der vier auf dem Yijing basierenden Charakterprofildatensätze kombiniert wird. Diese Vorgehensweise kann beispielsweise auch dann besonders vorteilhaft sein, wenn die körperliche Ebene eher separat ausgewertet werden soll, insbesondere erst dann mit den anderen Ebenen kombiniert werden soll, wenn diese bereits mittels des Astrologie-Ansatzes zusammengeführt wurden.

Gemäß einer Ausführungsform basieren vier der eigenständigen Charakterprofildatensätze auf dem Yijing und liefern jeweils ein Codeset, wobei ein weiterer eigenständiger Charakterprofildatensatz auf einem/dem Numerologie-Ansatz basiert und einen weiterer Codeset liefert, welcher mit den Codesets der vier auf dem Yijing basierenden Charakterprofildatensätze kombiniert wird. Dies ermöglicht nicht zuletzt auch die Schaffung einer möglichst breiten Informationsgrundlage für die Auswertung des Persönlichkeitsprofils. Vorteilhaft fokussieren die Yijing-basierten Codes der erfindungsgemäßen Codeauswahl dabei auf jeweils eine der folgenden Ebenen: emotional, mental, körperlich, transzendent.

Gemäß einer Ausführungsform werden 64 codesetübergreifende Persönlichkeitseigenschafts-Codes generiert. Dies ermöglicht nicht zuletzt auch die Beibehaltung einer vorbekannten Systematik, zumindest zu einem gewissen Grade, und erleichtert auch eine Plausibilitätsprüfung.

Bezüglich der Implementierung des hier beschriebenen Numerologie-Ansatzes, welcher nicht auf der 64er-Systematik beruht, lässt sich erwähnen, dass dieser mit den Zahlen von 0-9 zehn Codes aus jeweils zwei Methoden (also 20 Codes) liefert, welche wahlweise noch miteinander kombiniert werden könn(t)en. Im Rahmen der vorliegenden Erfindung hat sich gezeigt, dass es zielführend ist, nur diese 20 Codes und nicht auch deren Kombination(en) zu nutzen, insbesondere indem Überschneidungen bzw. Ergänzungen mit dem astrologischen Ansatz ausgewertet werden, so dass der Numerologie-Ansatz das 64er-System der anderen Ansätze ergänzen und es zu einer höheren Präzision führen kann.

Es hat sich gezeigt, dass die Implementierung der einzelnen Ansätze auch derart erfolgen kann, dass die Numerologie-Codes auch als nur optionale Ergänzung der Persönlichkeitsprofilbeschreibung lediglich optional ausgegeben werden könn(t)en, z.B. ausgegeben in Abhängigkeit vom Adressaten, z.B. auch in Hinblick auf einen besseren Schutz des Individuums oder aus ethischen Gründen. Beispiel: Ein Numerologie-Code spiegelt wieder, dass ein Individuum besonders anfällig ist für Migräne. Die Codeausgabe kann dann (je nach Adressat) basierend auf ausgeblendetem Numerologie-Code erfolgen, z.B. zugunsten eines Astrologie-Codes, welcher nicht so stark auf einen hohen körperlichen Detailgrad ausgerichtet ist, sondern eher nur eine körperliche Ausstrahlung beschreiben (vergleiche hierzu die hier an anderer Stelle beschriebenen optional mehrstufige Ausgabe der Persönlichkeitsprofilbeschreibung in unterschiedlichen Detaillierungsgraden).

Gemäß einer Ausführungsform werden 64 codesetübergreifende Persönlichkeitseigenschafts-Codes generiert, aus denen neun Codes als oberbegriffsartige Hauptcodes definiert sind, basierend auf welchen eine Ausgabe des Persönlichkeitsprofils im Umfang eines Fließtextes von ca. einer halben DinA4-Seite, maximal einer ganzen DinA4-Seite realisierbar ist (insbesondere bei standardmäßiger Schriftgröße von 12 Punkten und 1-1,5fachem Zeilenabstand). Dies ermöglicht nicht zuletzt auch eine besonders effektive und nutzerfreundliche Datenaufbereitung und Informationsausgabe bzw. Visualisierung.

Dabei können insbesondere im Rahmen der auf dem Astrologie-Ansatz basierenden Betrachtung über die individuenspezifischen Kennwerte individuenspezifisch astrologische Koordinaten abgerufen oder bestimmt werden, die eine mit der entsprechenden Zeit und dem entsprechenden Ort korrelierende individuenspezifische Planetenkonstellation kennzeichnen (wobei die jeweilige korrelierende Planetenkonstellation bevorzugt bereits als Bestandsdaten hinterlegt ist und aus einer/der Datenbank abrufbar ist), wobei das Persönlichkeitsprofil bzw. die Datengrundlage durch wenigstens einen (entsprechenden) astrologischen individuenspezifischen Parameter ergänzt werden kann, welcher aus diesen astrologischen Koordinaten erzeugt wird. Durch die Verknüpfung von Astrologie-Ansatz und den hier beschriebenen weiteren Ansätzen können ansatzübergreifend Codes generiert werden, insbesondere 64 Codes, welche jeweils für eine Persönlichkeitseigenschaft stehen.

Gemäß einer Ausführungsform werden im Rahmen des Numerologie-Ansatzes erste Codes basierend auf einer Methode nach Goodman ermittelt bzw. ausgewählt und/oder durch Variablen und Modalitäten charakterisiert. Zweite Codes werden vorteilhaft basierend auf einer Methode nach Hasler ermittelt bzw. ausgewählt und/oder durch Archetype, Hauptzustände und Nebenzustände charakterisiert. Dabei kann die absolute Anzahl von ermittelten oder vergebbaren codesetübergreifenden Persönlichkeitseigenschafts-Codes der absoluten Anzahl von ermittelten oder ermittelbaren Identifizierungs-/Charaktermerkmalen entsprechen, wobei exakt ein codesetübergreifender Persönlichkeitseigenschafts-Code mit exakt nur einem spezifischen Identifizierungs-/Charaktermerkmal korreliert.

Vorteilhaft ist/wird die Mehrzahl von codesetübergreifenden Persönlichkeitseigenschafts-Codes aus einer Mehrzahl von eigenständigen Codesets, insbesondere wenigstens fünf eigenständige Codesets gebildet. Weiter vorteilhaft können aus der Datengrundlage (bzw. aus den astrologischen Koordinaten und/oder wenigstens einem astrologischen Parameter) die Mehrzahl von codesetübergreifenden Persönlichkeitseigenschafts-Codes erzeugt/ermittelt werden, indem eine Mehrzahl von eigenständigen Codesets, insbesondere wenigstens fünf eigenständige Codesets genutzt werden, insbesondere Codesets betreffend die folgenden Charakterprofildatensätze: Human Design-Profildaten, Genekeys-Profildaten, Numerologie-Profildaten, Bazi Suan Ming-Profildaten, astrologische Profildaten. Anders ausgedrückt: Es kann eine Genekeys-Codeset mit einem Human Design-Codeset und/oder mit einem Numerologie-Codeset und/oder mit einem Bazi Suan Ming-Codeset und/oder mit einem astrologischen Codeset kombiniert werden. Dies erweitert auch beträchtlich die nutzbare/auswertbare Datengrundlage und kann Lücken oder Unzulänglichkeiten in den einzelnen vorbekannten Ansätzen zur Charakterprofildefinition schließen bzw. überwinden.

Gemäß einer Ausführungsform werden für ein spezifisches Individuum mehrere Codes eigenständiger Codesets miteinander kombiniert zum Erweitern der individuenspezifischen Datengrundlage bzw. zum Erweitern/Konkretisieren des jeweiligen Persönlichkeitsprofils durch daraus generierte codesetübergreifende Persönlichkeitseigenschafts-Codes, insbesondere indem spezifische Codes für wenigstens die folgenden vier Charakterebenen generiert bzw. ausgewertet werden: emotionale Charakterebene, körperliche Charakterebene, mentale Charakterebene, transzendente Charakterebene. Dies liefert nicht zuletzt auch eine sehr tiefgehende detailreiche Datengrundlage.

Beispielsweise sind wenigstens 64 erste Codes und wenigstens fünf (5) zweite Codes vorgesehen; und/oder beispieslweise sind wenigstens sechs Variablen und zwei Modalitäten zur Charakterisierung der ersten Codes vorgesehen. Beispielsweise sind wenigstens zwölf (12) Archetypen und wenigstens neun (9) Hauptzuständen und wenigstens 36 Nebenzuständen zur Charakterisierung von zweiten Codes vorgesehen. Beispielsweise umfasst die Datengrundlage wenigstens 768 Identifizierungs-/Charaktermerkmale, wobei diese Identifizierungs-/Charaktermerkmale mittels der Codes individualisiert je Individuum zuordenbar sind (zumindest jeweils eine Teilauswahl davon, je nach Persönlichkeitsprofil).

Gemäß einer Ausführungsform wird die Datengrundlage des Persönlichkeitsprofils computergestützt/- implementiert weiter ergänzt, indem wenigstens fünf eigenständige Charakterprofildatensätze zum individuenspezifischen Erstellen/Generieren/Auswerten eines/des charakterprofildatensatzübergreifenden Persönlichkeitsprofils herangezogen und ausgewertet werden, insbesondere basierend auf charakterprofildatensatzspezifischen Codes, nämlich wenigstens die fünf folgenden eigenständigen Charakterprofildatensätze bzw. entsprechende Codes (insbesondere Codeauswahl): Human Design, Genekeys, Bazi Suan Ming, Numerologie, Astrologie; insbesondere in der hier genannten Reihenfolge. Dies begünstigt nicht zuletzt ein umfangreiches und gleichwohl zielgerichtetes Auswerten des jeweiligen Persönlichkeitsprofils und optional nachgelagerter Persönlichkeitsprofilvergleiche. Zwar kann die Reihenfolge zumindest einzelner der Ansätze auch variiert werden, es hat sich jedoch gezeigt, dass es besonders vorteilhaft ist, zunächst eine Codeauswahl aus den drei erstgenannten Ansätzen vorzunehmen, um daraufhin den Numerologie-Ansatz zu applizieren, und abschließend mittels des Astrologie-Ansatzes eine ebenenübergreifende Korrelation zu vollziehen.

Gemäß einer Ausführungsform ist/wird aus wenigstens fünf eigenständigen Charakterprofildatensätzen die folgende Codeauswahl getroffen: Human Design-Codes in der gesamten Verfügbarkeit, dreizehn Genekeys-Codes, zehn Bazi Suan Ming-Codes, zwanzig Numerologie-Codes, insbesondre in der gesamten Verfügbarkeit von 2x10 Codes (erste und zweite Codes), Astrologie-Codes in der gesamten Verfügbarkeit. Dies ermöglicht nicht zuletzt auch eine sehr komplexe und vielschichtige Betrachtung der Persönlichkeit, insbesondere in/auf wenigstens dreizehn (13) unterschiedlichen Ebenen, insbesondere auch dahingehend, dass die einzelnen Codes aus den einzelnen Ansätzen miteinander kompatibel sind und sich ergänzen anstatt sich zu widersprechen.

Gemäß einer Ausführungsform ist/wird jeder codesetübergreifende Persönlichkeitseigenschafts-Code mit einem einzigen Textbaustein korreliert und entsprechend in der Datenbank hinterlegt, wobei das Persönlichkeitsprofil in Ausgestaltung als lesbarer (Fließ-)Text computerautomatisiert generiert wird basierend auf der Gesamtheit der individuenspezifisch ermittelten codesetübergreifenden Persönlichkeitseigenschafts-Codes bzw. -Textbausteine. Dies ermöglicht nicht zuletzt auch das Generieren eines durch Menschen ohne technische Hilfsmittel auslesbaren (Fließ-)Textes oder einer Tabelle, welche/r das Persönlichkeitsprofil umfassend beschreibt. Hieran ist auch ersichtlich, dass zum einen codiert Rohdaten generiert bzw. ausgegeben werden können, zum anderen auch unmittelbar ein komplettes Persönlichkeitsprofil, insbesondere je nachdem, an welchen Adressaten die Daten zu übermitteln sind.

Gemäß einer Ausführungsform sind zumindest die folgenden individuenspezifischen Kennwerte über eine Benutzerschnittstelle eingebbar und daraufhin computerautomatisiert auswertbar, insbesondere zur Datenpflege einer/der durch die jeweils individuenspezifisch verwirklichten codesetübergreifenden Persönlichkeitseigenschafts-Codes gebildeten (Identitäts-)Matrix (Persönlichkeitsprofildatensatz in Matrix-Form): Geburtsdatum, Geburtszeit, Geburtsort (Kennwerte) sowie optional auch Name und/oder Profilbild. Dies begünstigt nicht zuletzt eine noch gezieltere Suche und Auswahl von geeigneten Individuen.

Gemäß einer Ausführungsform ist für den Abgleich der Persönlichkeitsprofile wenigstens ein Attribut (Priorisierungs-/Gewichtungsparameter) vorgebbar, über welches die Relevanz (Impact) wenigstens eines bzw. eines jeweiligen individuenspezifisch ermittelten codesetübergreifenden Persönlichkeitseigenschafts-Codes vordefiniert/vordefinierbar ist. Dies ermöglicht z.B. auch eine anwendungsspezifische Gewichtung, z.B. beim computergestützten Zusammenstellen von Teams für ganz bestimmte Tätigkeiten. Der Abgleich kann bevorzugt über die Benutzerschnittstelle erfolgen. Es ist erwähnenswert, dass gemäß einer beispielhaften Implementierung für das Matching bzw. für den hier beschriebenen Abgleich z.B. neun (9) bis 64 Eigenschaften ausgewählt werden können, die wahlweise unmittelbar mit entsprechenden codesetübergreifenden Persönlichkeitseigenschafts-Codes korreliert sind; wahlweise kann diese Attributierung auch durch entsprechende Vorschläge über die Benutzerschnittstelle erleichtert werden. Nach Attributierung erfolgt systemseitig ein Scoring, basierend auf welchem eine attributspezifische Bewertung bzw. Zuordnung erleichtert wird.

Gemäß einer Ausführungsform umfasst das Verfahren eine Verarbeitung wenigstens eines individuenspezifischen (eingegebenen oder abgerufenen) Kennwerts unter Bezugnahme auf Codes wenigstens eines Charakterprofildatensatzes oder Ansatzes aus der folgenden Gruppe: Genekeys-Ansatz, Human Design-Ansatz, Numerologie-Ansatz, Bazi Suan Ming-Ansatz, astrologischer Ansatz. Dies erleichtert nicht zuletzt auch eine Gewichtung der einzelnen Persönlichkeitsprofilmerkmale, insbesondere zwecks anwendungsbezogenem Abgleich von Persönlichkeitsprofilen.

Gemäß einer Ausführungsform erfolgt ein/der Abgleich der Persönlichkeitsprofile bzw. Persönlichkeitseigenschaften von wenigstens zwei Individuen basierend auf einem mittels der individuenspezifisch ermittelten codesetübergreifenden Persönlichkeitseigenschafts-Codes und wenigstens eines vorgebbaren Attributs (welches die Relevanz eines jeweiligen individuenspezifisch ermittelten Codes vordefiniert) erstellten anwendungsspezifisch gewichteten Klassifizierung, wobei die jeweils wenigstens zwei miteinander verglichenen Individuen basierend auf einer wenigstens drei- oder vierstufigen Klassifizierung, welche den Grad bzw. die Anzahl der verwirklichten/ermittelten/abgeglichenen übereinstimmenden Persönlichkeitseigenschaften abbildet, entweder einer gleichen Gruppe von Individuen zugeordnet werden (bzw. ein Individuum wird einem weiteren Individuum oder einer Gruppe weiterer Individuen zugewiesen) oder jeweils einer anderen Gruppe von Individuen zugewiesen oder als nicht miteinander vereinbar klassifiziert werden: 90-100% Vereinbarkeit/Zuweisung, 75-89% Vereinbarkeit/Zuweisung potentiell möglich, <75% Vereinbarkeit/Zuweisung potentiell zu verneinen, und optional <50% Zuweisung unmöglich (systemseitig unterbunden). Dies erleichtert nicht zuletzt die Auswahl und Zuordnung von in charakterlicher Hinsicht potentiell sehr wertvollen Teammitgliedern.

Gemäß einer Ausführungsform erfolgt ein/der Abgleich der Persönlichkeitsprofile bzw. Persönlichkeitseigenschaften von wenigstens drei Individuen, wobei wenigstens eines der abgeglichenen Individuen basierend auf einer mittels wenigstens eines anwendungsspezifisch vorgegebenen Attributs erstellten gewichteten Klassifizierung gegenüber wenigstens einem der anderen Individuen priorisiert zugewiesen wird, insbesondere auch dann wenn die Anzahl korrelierender individuenspezifisch ermittelter codesetübergreifender Persönlichkeitseigenschafts-Codes gleich groß ist. Dies ermöglicht nicht zuletzt eine noch gezieltere Auswahl in Abhängigkeit von vordefinierbaren Kriterien.

Gemäß einer Ausführungsform wird für einen/den Abgleich von wenigstens zwei Persönlichkeitsprofilen wenigstens ein Attribut (Priorisierungs-/Gewichtungsparameter) vorgegeben, über welches die Relevanz (Impact) eines jeweiligen codesetübergreifenden Persönlichkeitseigenschafts-Codes für den jeweiligen Abgleich der Persönlichkeitsprofile vordefiniert wird. Dies ermöglicht nicht zuletzt eine sehr fokussierte Auswahl von Individuen im codesetübergreifenden Datensatz, also auf der Grundlage der erfindungsgemäß zusätzlichen Informationen, die durch gemeinsame Auswertung der einzelnen (insbesondere fünf) Charakterprofildatensätze generiert werden können. Vorteilhaft wird dabei nur auf die erfindungsgemäß generierten charakterprofildatensatzübergreifenden Codes zurückgegriffen. Wahlweise kann die Attributierung auch basierend auf jeweiligen charakterprofildatensatzspezifischen Codes vorgenommen werden.

Gemäß einer Ausführungsform werden für jedes Individuum alle ermittelten oder verfügbaren Codes bestimmt oder ausgelesen und mit allen ermittelten oder verfügbaren Codes des wenigstens einen weiteren Individuums basierend auf den jeweils daraus individuenspezifisch generierten codesetübergreifenden Persönlichkeitseigenschafts-Codes verglichen/abgeglichen, wobei eine Zuweisung von Individuen zu derselben Gruppe bevorzugt basierend auf wenigstens einem abgleichspezifisch vorgebbaren Attribut zum Einstellen des Impact eines jeweiligen Persönlichkeitseigenschafts-Codes bzw. einer jeweiligen Persönlichkeitseigenschaft erfolgt. Dies ermöglicht auch einen besonders umfangreichen Abgleich von Charaktermerkmalen, auch denjenigen, die beim jeweiligen Individuum gegebenenfalls nur sehr schwach oder gar nicht ausgeprägt sind.

Gemäß einer Ausführungsform basieren eine Mehrzahl der eigenständigen Charakterprofildatensätze auf dem Yijing, wobei ein erster Charakterprofildatensatz auf dem Human Design-Ansatz basiert und einen ersten Codeset liefert, welcher eine mentale Charakterebene beschreibt, wobei aus diesem Codeset alle verfügbare Codes entnommen bzw. genutzt werden, und wobei aus wenigstens einem der weiteren eigenständigen Charakterprofildatensätzen jeweils nur eine Codeauswahl generiert bzw. verwendet wird. Dies erleichtert nicht zuletzt auch eine Kombination bzw. Korrelation der einzelnen Codes aus den unterschiedlichen Ansätzen miteinander. Es hat sich auch gezeigt, dass es nicht zuletzt auch systematisch besonders vorteilhaft ist, ausgehend von den Human Design-Codes als zweite Codeauswahl Codes aus dem Genekeys-Ansatz betreffend die emotionale Ebene zu nutzen, um daraufhin die transzendente Ebene und die körperliche Ebene zu betrachten.

Die zuvor genannte Aufgabe wird auch gelöst durch ein Computerprogrammprodukt umfassend Befehle, die bei Ausführung des Computerprogrammproduktes auf einem Computer diesen dazu veranlassen, ein Verfahren gemäß der vorliegenden Offenbarung auf dem Computer auszuführen; wobei das Computerprogramm bevorzugt auch eingerichtet ist zum Abgleichen der Persönlichkeitsprofile von wenigstens zwei Individuen unter Bezugnahme sowohl auf die ersten als auch auf die zweiten Codes des jeweiligen Individuums. Dies ermöglicht basierend auf den zuvor genannten Vorteilen auch eine vorteilhaft einfache Implementierung z.B. auf einem Smartphone, insbesondere im Rahmen einer Applikation (App).

Vorteilhaft ist das Computerprogrammprodukt auch eingerichtet zum Erstellen eines Persönlichkeitsprofils wenigstens eines Individuums basierend auf ersten Identifizierungsmerkmalen definiert durch erste Codes, die auf Variablen und Modalitäten basieren, und basierend auf zweiten Identifizierungsmerkmalen definiert durch zweite Codes, die auf Archetypen und Hauptzuständen und Nebenzuständen basieren.

Die zuvor genannte Aufgabe wird auch gelöst durch eine Datenbank mit wenigstens einer darin hinterlegten und computerautomatisiert aufrufbaren und abgleichbaren (Identitäts-)Matrix, in welcher individuenspezifisch eine Vielzahl von Codes jeweils charakterisierend eine einzige Persönlichkeitseigenschaft hinterlegt sind, wobei die Datenbank eingerichtet ist zur Implementierung eines Verfahren gemäß der vorliegenden Offenbarung zum Abgleichen der Persönlichkeitseigenschaften und zum Generieren von Gruppenempfehlungen betreffend miteinander harmonisierender Individuen, insbesondere mittels eines Computerprogrammproduktes gemäß der vorliegenden Offenbarung insbesondere basierend auf vordefinierbaren Attributen bzw.

Gewichtungsparametern betreffend wenigstens eine Persönlichkeitseigenschaft. Hierdurch lassen sich zuvor genannte Vorteile realisieren, insbesondere in Hinblick auf eine vorteilhafte Bereitstellung der für die hier beschriebene Auswertung bzw. für das Abgleichen von Persönlichkeitsprofilen heranzuziehenden Daten. Dabei sind abgesehen von Internetzugang auch keine besonderen Systemanforderungen erforderlich.

Die zuvor genannte Aufgabe wird auch gelöst durch eine Benutzerschnittstelle gemäß dem entsprechenden nebengeordneten Anspruch, nämlich durch eine Benutzerschnittstelle mit einer Anzeige-/Bedienmaske eingerichtet zum Eingeben wenigstens eines individuenspezifischen Kennwerts zum Erstellen eines Persönlichkeitsprofils wenigstens eines Individuums, insbesondere zum Abgleichen des Persönlichkeitsprofils mit dem Persönlichkeitsprofil wenigstens eines weiteren Individuums, wobei die Benutzerschnittstelle eingerichtet ist zum Einpflegen zumindest der folgenden individuenspezifischen Kennwerte: Geburtsdatum, Geburtszeit, Geburtsort; und wahlweise können auch Name und/oder Profilbild eingegeben werden; Erfindungsgemäß ist die Benutzerschnittstelle ferner eingerichtet zum Ausgeben einer Mehrzahl von Persönlichkeitseigenschafts-Codes für das jeweilige Individuum, insbesondere mit den Codes in übersetzter Ausgestaltung als durch das Individuum lesbare Textbausteine, wobei die Persönlichkeitseigenschafts-Codes unter Bezugnahme auf eine Mehrzahl von eigenständigen Charakterprofildatensätzen erzeugt sind, insbesondere wenigstens fünf eigenständige Charakterprofildatensätze, insbesondere Charakterprofildatensätze betreffend die folgenden Charakterprofile: Human Design-Profildaten, Genekeys-Profildaten, Numerologie-Profildaten, Bazi Suan Ming-Profildaten, Astrologie-Profildaten. Hierdurch ergeben sich zuvor genannte Vorteile, insbesondere in Hinblick auf hohe Praxistauglichkeit und möglichst unkomplizierte Anwendbarkeit des hier beschriebenen Verfahrens und auch hinsichtlich guter Verfügbarkeit der erfindungsgemäß erzielbaren Ergebnisse.

Gemäß einer Ausführungsform sind die Codes unter Bezugnahme auf die astrologischen Koordinaten erzeugt/ermittelt sind und jeweils durch wenigstens ein Merkmal aus der folgenden Gruppe charakterisiert und voneinander unterschieden/abgegrenzt werden: Variable, Modalität, Archetyp, Hauptzustand, Nebenzustand, Identifizierungs-/Charaktermerkmal. Dies ermöglicht auch eine noch spezifischere Analyse.

Gemäß einer Ausführungsform ist die Benutzerschnittstelle ferner eingerichtet zum Ausgeben einer Auswahl wenigstens eines Individuums aus einer Gruppe von Individuen, bezüglich welcher die Codes zum Abgleich der Persönlichkeitsprofile der Individuen verglichen wurden. Dies erleichtert nicht zuletzt auch die Teamauswahl.

Die Benutzerschnittstelle kann vorteilhaft eingerichtet sein zum Ausgeben (bzw. sinngemäß zum Visualisieren) des Persönlichkeitsprofils basierend auf wenigstens einem astrologischen Parameter, welcher aus astrologischen Koordinaten erzeugt wird, die über die entsprechenden individuenspezifischen Kennwerte bestimmt werden können und eine mit der entsprechenden Zeit und dem entsprechenden Ort korrelierende Planetenkonstellation charakterisieren.

Über die Benutzerschnittstelle können z.B. auch Nachbearbeitungen und Aufbereitungen an den Daten vorgenommen werden, insbesondere adressatenspezifisch, also z.B. betreffend die Anrede im Rahmen der Persönlichkeitsbeschreibung ("Sie" oder "Du" oder "die Person X").

Vorteilhaft ist die Benutzerschnittstelle web-basiert (Internet) bzw. web-basiert übertragbar/visualisierbar und mittels herkömmlicher Endgeräte oder Computer nutzbar.

Gemäß einem Ausführungsbeispiel ist die Benutzerschnittstelle eingerichtet zum Abfragen wenigstens eines wenigstens eine Persönlichkeitseigenschaft priorisierenden Attributs. Dies erleichtert nicht zuletzt auch eine z.B. themenbezogene Priorisierung von Charaktermerkmalen, so dass auch die Suche nach für spezifische Projekte oder Aufgaben besonders geeigneten Individuen erleichtert wird. Vorteilhaft ist die Benutzerschnittstelle eingerichtet zum Abfragen eines Gewichtungsfaktors wenigstens eines wenigstens eine Persönlichkeitseigenschaft priorisierenden Attributs. Dies ermöglicht auch noch, eine Relevanz des jeweiligen Charaktermerkmals zu verstärken, insbesondere im Sinne eines vorherrschenden oder dominierenden Charakterzuges.

Gemäß einem Ausführungsbeispiel ist die Benutzerschnittstelle eingerichtet zum Abfragen eines (Kompatibilitäts-)Schwellwerts hinsichtlich eines Grades einer Übereinstimmung von codesetübergreifenden Codes (insbesondere gemäß einer prozentualen Anzahl der ermittelten übereinstimmenden Persönlichkeitseigenschaften) bzw. Charaktermerkmalen. Anders ausgedrückt: Ein jeweiliger Nutzer kann vorgeben, ob Individuen auch dann gelistet werden sollen, wenn z.B. nur 70% der vordefinierten Kriterien bzw. Charaktereigenschaften übereinstimmen. Dies erleichtert nicht zuletzt auch die Suche und Auswahl in einem vergleichsweise großen Pool von potentiell geeigneten Individuen. Als "Kompatibilitätsschwellwert" ist dabei insbesondere ein prozentualer Schwellwert, z.B. 80%, zu verstehen, ab welchem systemseitig (computergestützt) ein Individuum als potentiell geeignet vorgeschlagen wird.

Gemäß einem Ausführungsbeispiel ist die Benutzerschnittstelle eingerichtet zum Einpflegen und Ausgeben eines Profilbildes und/oder eines Namens des jeweiligen Individuums. Dies ermöglicht nicht zuletzt eine noch einfachere Zuordnung/Identifizierung von Individuen ausgehend von einem computergestützten Vorschlag, z.B. im Rahmen von Bewerbungsgesprächen. Vorteilhaft ist die Benutzerschnittstelle eingerichtet zum Ausgeben einer Team-Zusammensetzung umfassend eine Vielzahl von Individuen mit jeweils aufeinander abgestimmten Persönlichkeitsprofilen, insbesondere in Abhängigkeit von vordefinierbaren über die Benutzerschnittstelle eingebbaren Attributen für die aufeinander abzustimmenden Persönlichkeitsprofile. Dies vereinfacht nicht zuletzt auch das Zusammenstellen von z.B. projekt- oder aufgabenbezogenen Teams. Vorteilhaft ist die Benutzerschnittstelle ferner eingerichtet zum Visualisieren einer systemseitig basierend auf dem Abgleich der Persönlichkeitsprofile empfohlenen Gruppenzusammensetzung aus wenigstens drei Individuen. Dies ermöglicht auch die Erstellung von Zukunftsszenarien für potentiell schlagkräftige Team, z.B. innerbetrieblich im Rahmen von Beförderungs- oder Jobrotation-Fallstudien.

Gemäß einem Ausführungsbeispiel ist die Benutzerschnittstelle eingerichtet zum Ausgeben von einer Auswahl von neun Persönlichkeitseigenschafts-Hauptcodes in einer 3x3-Visualisierung, insbesondere gemäß einem Puzzle mit neun Teilen oder gemäß einer 3x3-Martix, insbesondere bei farblicher Hervorhebung, insbesondere mit einer/der Darstellung der mentalen Ebene oberhalb von einer/der Darstellung der emotionalen Ebene oberhalb von einer/der Darstellung der körperlichen Ebene, insbesondere mit einer/der Darstellung der transzendenten Ebene im vertikal-mittigen Bereich. Dies erleichtert nicht zuletzt auch eine Art Standardisierung der Persönlichkeitsprofilbeschreibung und ermöglicht einen noch einfacheren Vergleich/Abgleich, auch in visueller Hinsicht, was nicht zuletzt z.B. im Kontext mit einer Bearbeitung einer Vielzahl von Bewerbungen die Arbeit z.B. im Personalmanagement deutlich erleichtert.

Gemäß einem Ausführungsbeispiel ist die Benutzerschnittstelle eingerichtet zum Ausgeben der Information von 64 codesetübergreifenden Persönlichkeitseigenschafts-Codes auf wenigstens zwei unterschiedlichen Detaillierungsstufen, insbesondere über ein spezifisches Eingabefeld für den Detaillierungsgrad, von denen neun Codes als oberbegriffsartige Hauptcodes definiert sind, basierend auf welchen eine Ausgabe des Persönlichkeitsprofils im Umfang eines Fließtextes von ca. einer halben DinA4-Seite, maximal einer ganzen DinA4-Seite realisierbar ist (insbesondere bei standardmäßiger Schriftgröße von 12 Punkten und 1-1,5fachem Zeilenabstand). Dies ermöglicht nicht zuletzt auch eine graduelle Analyse bzw. ein abgestuftes Vorgehen, z.B. indem ein detaillierter Abgleich unter Individuen nur innerhalb einer bereits vorausgewählten Gruppe erfolgt.

Es ist zu verstehen, dass das Persönlichkeit jeweils vollständig erstellt/generiert wird; lediglich die Ausgabe bzw. Visualisierung der Information mag wahlweise auf graduelle bzw. mehr oder weniger detaillierte Art und Weise erfolgen, je nach Nutzerpräferenz.

Die zuvor genannte Aufgabe wird auch gelöst durch Verwendung einer in einer Datenbank hinterlegten (Identitäts-)Matrix mit individuenspezifisch hinterlegten codesetübergreifenden Persönlichkeitseigenschafts-Codes jeweils charakterisierend eine Persönlichkeitseigenschaft zum computerautomatisierten/-implementierten Aufrufen und Abgleichen von Persönlichkeitsprofilen von einer Vielzahl von Individuen hinsichtlich wenigstens eines vordefinierten/vordefinierbaren Soll-Persönlichkeitsprofils, wobei ein Abgleich von zumindest den folgenden Persönlichkeitseigenschafts-Codes erfolgt: Human Design-Codes, Genekeys-Codes, Numerologie-Codes, Bazi Suan Ming-Codes, Astrologie-Codes; insbesondere mit dem Soll-Persönlichkeitsprofil erstellt in Abhängigkeit von den Persönlichkeitseigenschaften einer Mehrzahl von Persönlichkeitsprofilen bzw. Individuen einer bereits angelegten/erstellten Gruppe von Individuen mit jeweils individuell individuenspezifisch vordefiniertem/vordefinierbarem Soll-Persönlichkeitsprofil, insbesondere unter Anwendung eines Verfahrens gemäß der vorliegenden Offenbarung. Hierdurch lassen sich zuvor genannte Vorteile realisieren, insbesondere in Hinblick auf effizientes Datenmanagement und Bereitstellung einer vorteilhaft nutzbaren Datenbasis für die hier beschriebenen Schritte zum Identifizieren/Auswählen von geeigneten Individuen und/oder zum Konzipieren von vorteilhaft harmonisierenden Gruppenzusammensetzungen.

Zusammenfassung: Bei der Bestimmung eines Persönlichkeitsprofils besteht Interesse an einer möglichst breiten Datengrundlage, insbesondere um eine möglichst belastbare Charakterisierung vornehmen zu können. Bereitgestellt wird ein Verfahren zum Erstellen und Abgleichen von Persönlichkeitsprofilen, wobei dafür eine Datengrundlage basierend auf zumindest den folgenden individuenspezifischen Kennwerten erstellt oder abgerufen sowie ausgewertet wird: Geburtsdatum, Geburtszeit, Geburtsort, wahlweise auch Name und/oder Profilbild; Erfindungsgemäß wird die Datengrundlage ergänzt, indem eine Mehrzahl von eigenständigen Charakterprofildatensätzen zum individuenspezifischen Erstellen/Generieren eines charakterprofildatensatzübergreifenden Persönlichkeitsprofils herangezogen werden, insbesondere wenigstens fünf eigenständige Charakterprofildatensätze, insbesondere Charakterprofildatensätze betreffend die folgenden Charakterprofile: Human Design-Profil(daten), Genekeys-Profil(daten), Numerologie-Profil(daten), Bazi Suan Ming-Profil(daten), Astrologie-Profil(daten). Hierdurch kann der Umfang der auswertbaren Daten erweitert werden, und das Abgleichen kann hinsichtlich zahlreicher Kriterien und bei hoher Datengüte erfolgen. Die Erfindung betrifft ferner entsprechende Vorrichtungen bzw. Systemkomponenten und Verwendungen.

### KURZE BESCHREIBUNG DER FIGUREN

In den nachfolgenden Zeichnungsfiguren wird die Erfindung noch näher beschrieben, wobei für Bezugszeichen, die nicht explizit in einer jeweiligen Zeichnungsfigur beschrieben werden, auf die anderen Zeichnungsfiguren verwiesen wird. Es zeigen jeweils in schematischer Darstellung:
- **Figur 1**: Schritte eines Verfahrens gemäß einem Ausführungsbeispiel;
- **Figur 2**: Komponenten einer IT-Infrastruktur zur Realisierung eines Verfahrens gemäß Ausführungsbeispielen;
- **Figur 3**: einen Ausgangspunkt zum Generieren eines charakterprofildatensatzübergreifenden Persönlichkeitsprofils gemäß Ausführungsbeispielen,
- **Figur 4**: Schritte eines Verfahrens gemäß einem weiteren Ausführungsbeispiel;
- **Figur 5**: Schritte zur Codegenerierung bei einem Verfahren gemäß Ausführungsbeispielen;
- **Figur 6**: Schritte eines Verfahrens gemäß Ausführungsbeispielen, wobei das Verfahren auch ein Attributieren und Abgleichen umfasst;
- **Figur 7**: eine Veranschaulichung einer Abhängigkeit der einzelnen Ansätze sowie einer bevorzugten Reihenfolge zur Generierung der charakterprofildatensatzübergreifenden Daten bzw. Codes oder Textbausteine gemäß Ausführungsbeispielen;
- **Figur 8**: eine Veranschaulichung einer schrittweisen Code- und Fließtextgenerierung ausgehend von vorbekannten Ansätzen zum Generieren eines charakterprofildatensatzübergreifenden Persönlichkeitsprofils gemäß Ausführungsbeispielen;
- **Figur 9**: eine beispielhafte Veranschaulichung einer grafischen Visualisierung eines charakterprofildatensatzübergreifenden Persönlichkeitsprofils insbesondere in Ergänzung zu einer textbasierten Beschreibung des jeweiligen Persönlichkeitsprofils basierend auf den gemäß Ausführungsbeispielen generierten Codes;

### DETAILLIERTE BESCHREIBUNG DER FIGUREN

Die Erfindung wird zunächst unter allgemeiner Bezugnahme auf alle Bezugsziffern und Figuren erläutert. Besonderheiten oder Einzelaspekte oder in der jeweiligen Figur gut sichtbare/darstellbare Aspekte der vorliegenden Erfindung werden individuell im Zusammenhang mit der jeweiligen Figur thematisiert.

Bereitgestellt wird eine Datenbank 1, die über einen Server 2 zugänglich ist, und in welcher individuenspezifische Charakterprofildatensätze 3 für jeweils ein Charakterprofil insbesondere aus der folgenden Gruppe hinterlegt sind bzw. werden können: Human Design-Profil(daten), Genekeys-Profil(daten), Numerologie-Profil(daten), Bazi Suan Ming-Profil(daten), Astrologie-Profil(daten). Für das jeweilige Individuum können zumindest ein Human Design-Charakterprofildatensatz 3a, ein Genekeys-Charakterprofildatensatz 3b, ein Bazi Suan Ming-Charakterprofildatensatz 3c, ein Numerologie-Charakterprofildatensatz 3d und ein Astrologie-Charakterprofildatensatz 3e hinterlegt sein bzw. generiert werden, wobei jeder Charakterprofildatensatz basierend auf einem eigenständigen Codeset 3.1 mit einer Vielzahl von Codes 3.11, korrelierbar/korreliert mit jeweils einem einzelnen Charaktermerkmal, ein mehr oder weniger vollständiges Charakterprofil des Individuums zeichnen/abbilden kann, insbesondere jeweils mit spezifischer Gewichtung, z.B. vornehmlich hinsichtlich einer/der transzendenten oder emotionalen oder körperlichen oder mentalen Charakterebene.

Ausgehend von diesen wenigstens fünf Datensätzen kann individuenspezifisch ein Persönlichkeitsprofildatensatz 10 bzw. eine entsprechende Datengrundlage generiert werden, insbesondere in Matrixkonfiguration, welche/r durch codesetübergreifende Persönlichkeitseigenschafts-Codes 10.11 basiert, welche aus diesen Datensätzen generiert werden können, wobei diese Codes 10.11 jeweils mit einem einzelnen Charaktermerkmal korrelierbar/korreliert sind, so dass ein individuenspezifische codesetübergreifendes Persönlichkeitsprofil 10i aus diesen codesetübergreifenden Persönlichkeitseigenschafts-Codes generiert werden kann, in der Art einer Codeauswahl 10.1 aus der Mehrzahl von eigenständigen Codesets 3.1 der vorbekannten Ansätze 3.

Wahlweise kann (codesetübergreifend) ein gewichtetes Persönlichkeitsprofil 10i' mit Scoring generiert werden, insbesondere indem wenigstens ein Attribut 14 (Priorisierungs-/Gewichtungsparameter) zur Vorgabe einer Relevanz (Impact) eines jeweiligen codesetübergreifenden Persönlichkeitseigenschafts-Codes appliziert wird. Das jeweilige Attribut kann z.B. themenspezifisch oder auftraggeberspezifisch vorgegeben sein/werden.

Über eine Benutzerschnittstelle 20, z.B. bereitgestellt über eine App und mittels eines Endgeräts (z.B. Smartphone) 100, kann über ein erstes Eingabefeld 21 ein individuenspezifischer Kennwert i, insbesondere Geburtsdatum, Geburtszeit, Geburtsort, wahlweise auch Name, Spitzname und/oder Profilbild (frontal), durch einen Nutzer bzw. durch das jeweilige Individuum I eingegeben werden. Über ein zweites Eingabefeld 22 kann wenigstens ein Attribut für einen gewichteten Abgleich von Persönlichkeitsmerkmalen appliziert bzw. vorgegeben werden, beispielsweise indem systemseitig bis zu 64 Eigenschaften über die Benutzerschnittstelle vorgeschlagen werden, die wahlweise unmittelbar mit entsprechenden codesetübergreifenden Persönlichkeitseigenschafts-Codes korreliert sind. Der Nutzer muss dann lediglich die gewünschte Attributierung bestätigen; so kann auch eine nichtzuordenbare Eingabe vermieden werden. Über ein drittes Eingabefeld 23 kann der Detaillierungsgrad (insbesondere entweder kurz oder detailliert) der Informationsausgabe vorgebeben werden, insbesondere wahlweise die in allen (vorteilhaft 64) generierten Codes enthaltene Information oder die in nur neun (9) Hauptcodes unter diesen Codes enthaltene Information, letztere wahlweise auch in zwei unterschiedlichen Detaillierungsgraden, z.B. entweder auf nur einer halben oder einer ganzen DinA4-Seite. Eine Ausgabe des jeweiligen Persönlichkeitsprofils oder auch einer Matching-Empfehlung kann z.B. mittels Ausgabemitteln 25 erfolgen, insbesondere als Fließtext (visuell über wenigstens ein Anzeigefeld) und/oder als gesprochene/vorgelesen Information (akustisch mittels wenigstens eines Lautsprechers oder dergleichen).

Insbesondere im Zusammenhang mit der Benutzerschnittstelle zur Vorgabe des Detaillierungsgrades (insbesondere Feld 23) kann auch eine Authentifizierungshürde implementiert sein; anders ausgedrückt: spätestens dann wenn ein Nutzer eine größere Detailliertheit als die in den Hauptcodes enthaltene Kurzversion wünscht, ist eine Authentifizierung oder ein Einloggen (z.B. passwortgeschützt, oder sogar über Zwei-Faktor-Authentifizierung implementiert) erforderlich, so dass systemseitig erkannt und archiviert werden kann, wer wann welche Information über welche Person abgerufen hat.

Das erfindungsgemäße Verfahren kann insbesondere im Kontext folgender Schritte durchgeführt bzw. angewandt oder implementiert werden:
Schritt **S1** Dateneingabe insbesondere über Benutzerschnittstelle;
Schritt **S2** Erstellung Datengrundlage: Profilbestimmung basierend auf einer Mehrzahl von Ansätzen;
Schritt **S2.1** Erstellung Datengrundlage: Profilbestimmung basierend auf Human Design-Ansatz;
Schritt **S2.2** Erstellung Datengrundlage: Profilbestimmung basierend auf Genekeys-Ansatz;
Schritt **S2.3** Erstellung Datengrundlage: Profilbestimmung basierend auf Bazi Suan Ming-Ansatz;
Schritt **S2.4** Erstellung Datengrundlage: Profilbestimmung basierend auf Numerologie-Ansatz;
Schritt **S2.5** Erstellung Datengrundlage: Profilbestimmung basierend auf Astrologie-Ansatz;
Schritt **S2.x** charakterprofildatensatzspezifischer Codeoutput;
Schritt **S3** Auswertung Datengrundlage aus der Mehrzahl von Ansätzen;
Schritt **S3.1** Korrelieren von Textbausteinen mit Codes, bzw. Erstellen eines (Fließ-)Textes basierend auf vordefinierter Korrelation von codesetübergreifenden Persönlichkeitseigenschafts-Codes und Textbausteinen;
Schritt **S3.2** Ausgabe eines individuenspezifischen Persönlichkeitsprofils z.B. als (Fließ-)Text;
Schritt **S3.3** zusammengefasste Persönlichkeitsprofilbeschreibung basierend auf neun Hauptcodes
Schritt **S3.x** charakterprofildatensatzübergreifender Codeoutput;
Schritt **S2S3** iterative Codegenerierung;
Schritt **S4** Attributieren: Erstellen/Berücksichtigen von Attributen zur individuellen Gewichtung von Charaktermerkmalen, dadurch Generieren eines gewichteten Persönlichkeitsprofils (Scoring), insbesondere im Kontext einer Rangfolge mehrerer Individuen,
Schritt **S5** Abgleichen (Matching) der Persönlichkeitsprofile bzw. Persönlichkeitsprofildaten mehrerer Individuen (oder eines Individuums mit einem Anforderungskatalog), entweder mit oder ohne zugewiesenen Attributen;
Schritt **S6** Ausgabe von Gruppen zueinander korrelierter/korrelierbarer Individuen, wahlweise bei Einbezug einer abgestuften Klassifizierung durch Priorisierung der systemseitig stärker zueinander korrelierten Individuen insbesondere in Abhängigkeit von wenigstens einem Prozentanteils-Schwellwert betreffend die prozentuale Überdeckung gewünschter Charaktermerkmale;
Schritt **S6.1** Zuweisung eines Individuums zu einer Gruppe, oder Identifizierung eines/des Individuums als "geeignet", oder Schritt **S6.2** Markierung eines/des Individuums als "ungeeignet", insbesondere bezüglich wenigstens eines anwendungsspezifisch applizierten Attributs;
Schritt **S7** computergestütztes/-implementiertes Auswählen und Einladen/Kontaktieren von wenigstens einem Individuum bei Identifizierung des Individuums als "geeignet",

Es ist zu verstehen, dass Schritt S5 und/oder S6 auch ein Abgleich bzw. Zuweisen eines Persönlichkeitsprofils mit Suchkriterien oder Vorgaben einer ein bestimmtes Persönlichkeitsprofil suchenden Entität sein kann. Anders ausgedrückt: diese Schritte müssen nicht notwendigerweise für Gruppenkonstellationen implementiert sein, sondern können sich auch auf ein einzelnes Individuum beziehen.

Im Folgenden werden Details oder Besonderheiten der Erfindung unter Bezugnahmen auf einzelne Figuren bzw. Ausführungsbeispiele erläutert.

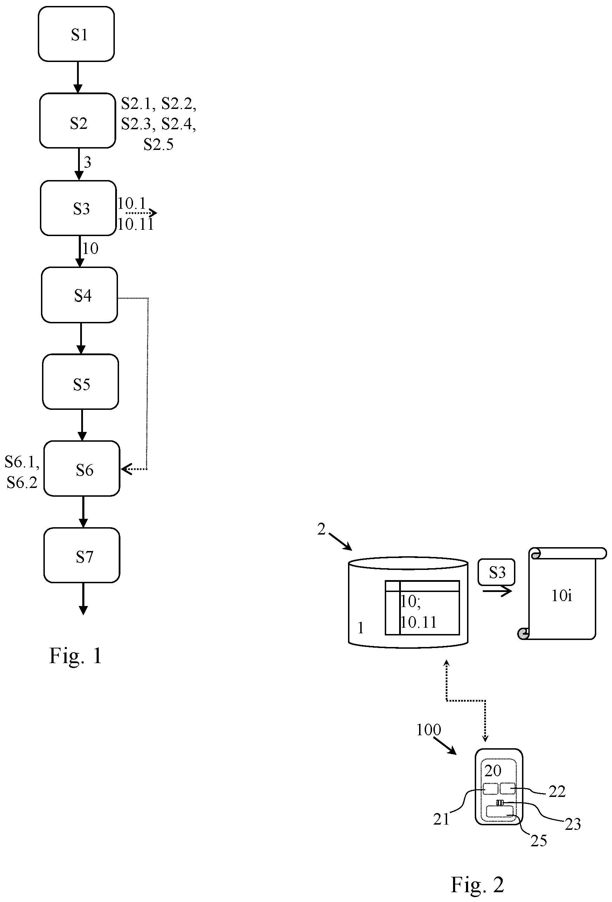

In **Fig. 1** ist ein beispielhafter Ablauf der hier beschriebenen Schritte skizziert; das Verfahren kann mit Schritt S3, Auswertung/Ausgabe des Persönlichkeitsprofils, bereits zum Abschluss gebracht werden; wahlweise erfolgt auch eine Auswahl eines einzelnen Individuums aus einem Pool mehrerer potentiell geeigneter Individuen, Schritt S5, oder es erfolgt eine Gruppenzuordnung, Schritt S6. Das Verfahren kann auch das zumindest teilweise computergestützte Kontaktieren/Einladen wenigstens eines Individuums umfassen, Schritt S7.

In **Fig. 2** sind vorrichtungstechnische bzw. miteinander kommunizierende/zusammenwirkende Komponenten einer IT-Infrastruktur zur Realisierung des hier beschriebenen Verfahrens skizziert. Vorteilhaft sind die erfindungsgemäß generierten codesetübergreifenden Codes 10.11 eines jeweiligen Individuums drahtlos zugänglich, insbesondere bei zugangsgeschützter/-beschränkter Archivierung, z.B. durch Passwortschutz oder sogar durch Zwei-Faktor-Authentifizierung.

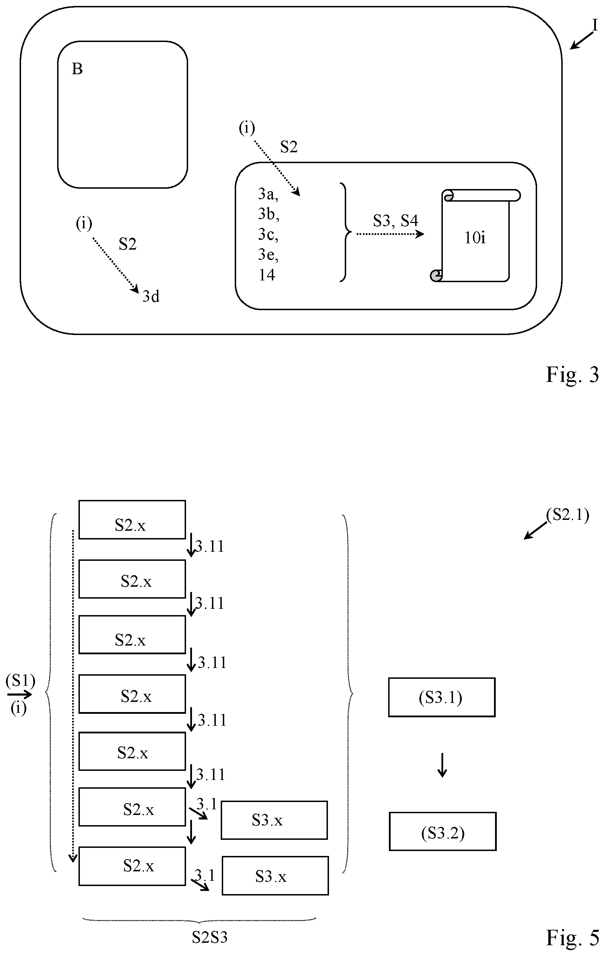

In **Fig. 3** ist der Ausgangspunkt der Analyse gezeigt. Für das hier beschriebene Verfahren werden vorteilhaft wenige persönliche Daten (Kennwerte) benötigt, insbesondere Geburtsdatum, Geburtszeit und Geburtsort; die Analyse kann sogar ausgehend von ausschließlich diesen persönlichen Daten realisiert werden bzw. implementiert sein. Diese Daten gehen in den jeweiligen Ansatz 3a bis 3e ein. Vorteilhaft kann im Rahmen der vorliegenden Erfindung dann eine Persönlichkeitsprofilbeschreibung 10i auf wahlweise nur einer halben DinA4-Seite erfolgen, insbesondere basierend auf neun (9) Hauptcodes - dies ist in Fig. 3 insofern veranschaulicht, als die dafür erforderliche Information als Fließtext z.B. auf einer Karteikarte dargestellt werden kann, also schnell erfassbar auf einem Computerbildschirm oder sogar auf einem Smartphone. Die ein jeweiliges Individuum I kennzeichnenden Daten können dabei optional auch ein Profilbild B (frontal) umfassen; ein Profilbild dient wie auch der Name jedoch lediglich der leichteren Zuordnung; diesbezügliche Daten sind also keine im Sinne der vorliegenden Erfindung ausgewerteten Kennwerte i.

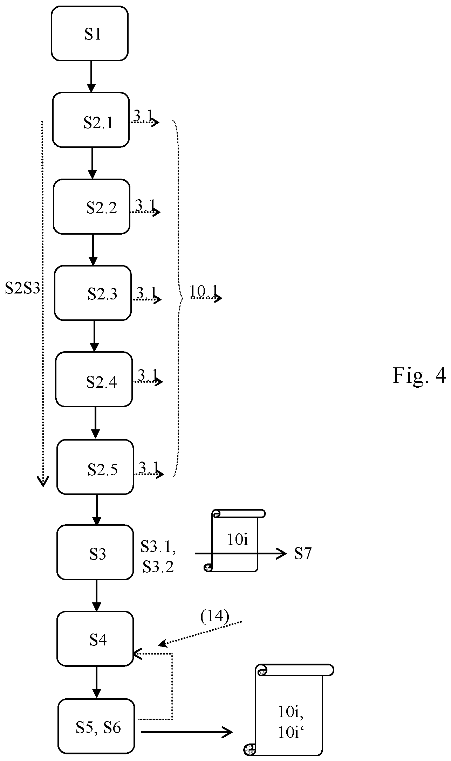

In **Fig. 4** ist der Zusammenhang der einzelnen Schritte zur Erzeugung der codesetübergreifenden Codeauswahl 10.1 zur codesetübergreifenden Definition des Persönlichkeitsprofils 10i veranschaulicht.

In **Fig. 5** ist ein Beispiel einer iterativen Codegenerierung (Schritt S2S3) ausgehend von einem der hier beschriebenen Ansätze skizziert, insbesondere ausgehend vom Human Design-Ansatz. Zunächst erfolgt eine Code-Generierung innerhalb des Rahmens des entsprechenden Ansatzes (Codes 3.11), insbesondere eine Code-Auswahl, daraufhin erfolgt eine Übergabe eines/des für diesen Ansatz spezifischen Codeset 3.1, zur weiteren Verarbeitung im Rahmen der erfindungsgemäßen gemeinsamen Betrachtung mehrerer Ansätze. Insofern kann dank des erfindungsgemäßen Ansatzes ein potentieller Konflikt bei einer Code-Migration verhindert werden, insbesondere dank einer Code-Auswahl aus den einzelnen Ansätzen, die auf den 64 Hexagrammen des Yijing basieren, und dank einer Kombination dieser Code-Auswahl mit Codes des Numerologie-Ansatzes.

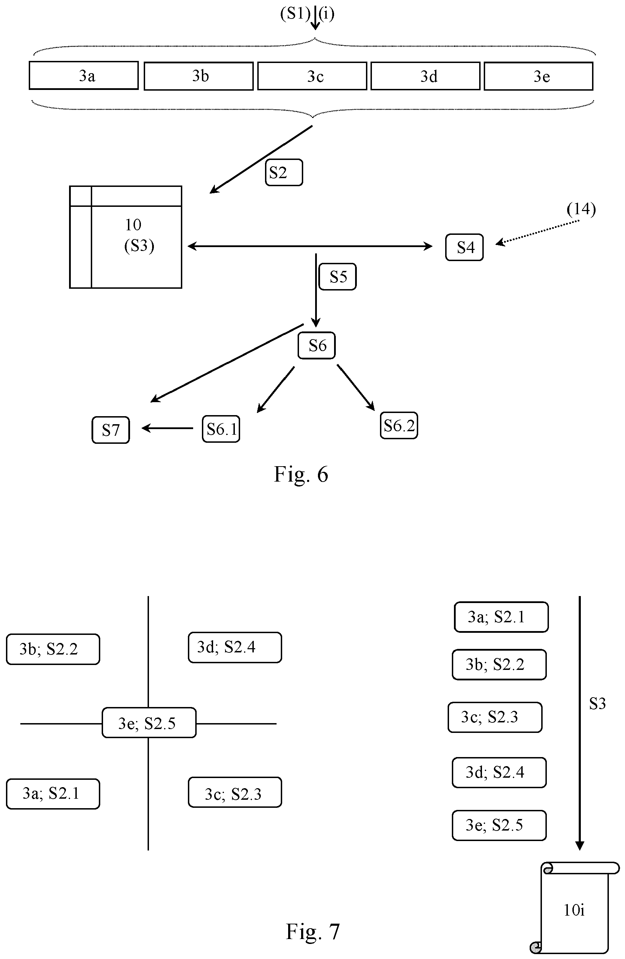

In **Fig. 6** ist eine Implementierung des Verfahrens skizziert, bei welcher auch eine Gruppenauswahl bzw. Gruppenzusammenstellung erfolgt, oder bei welcher ein einzelnes Individuum für eine bestimmte Gruppe oder für einen insbesondere im Rahmen der Attributierung vordefinierbaren Themenbereich als potentiell geeignet ausgewählt wird und computergestützt kontaktiert/eingeladen werden kann, Schritt S7, z.B. für ein Bewerbungsgespräch.

In **Fig. 7** ist überblicksartig der Zusammenhang zwischen dem Ausgangspunkt der einzelnen Ansätze 3a bis 3e und dem generierten kodifizierten und wahlweise als Fließtext ausgegebenen Persönlichkeitsprofil 10i visualisiert.

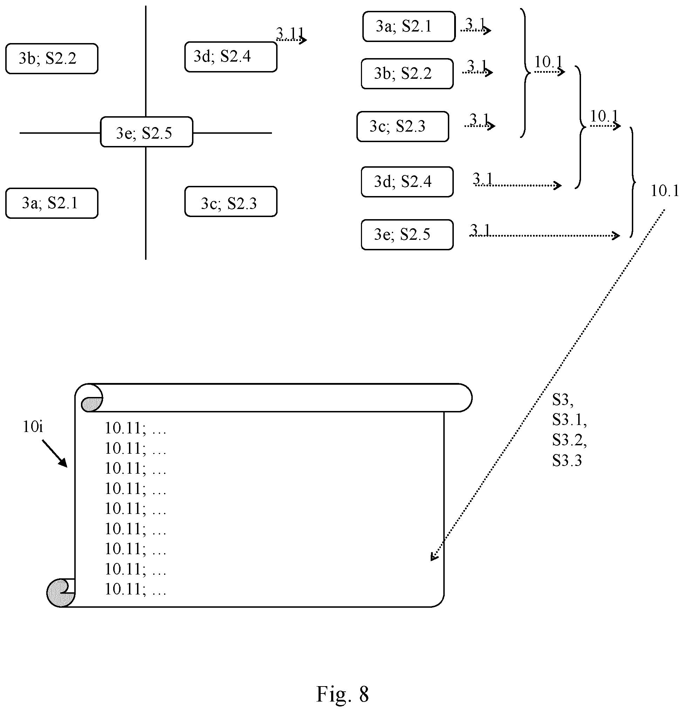

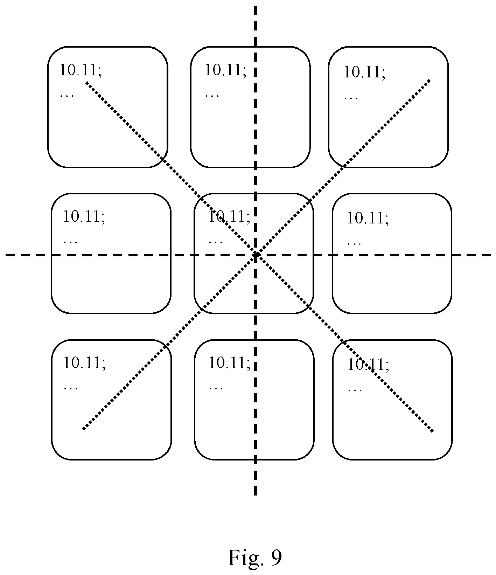

In **Fig. 8** ist veranschaulicht, wie ausgehend von den einzelnen Ansätzen 3a bis 3d die jeweilige Codeauswahl 3.1 umfassend ausgewählte Codes 3.11 erzeugt wird, um dann schrittweise eine finale Codeauswahl für die codesetübergreifende Codeauswahl 10.1 zu generieren, insbesondere 64 Codes, welche die Grundlage für entweder eine sehr ausführliche Persönlichkeitsprofilbeschreibung 10i bilden (Textbausteine werden basierend auf allen Codes der Codeauswahl 10.1 generiert), oder für eine überblickartige Persönlichkeitsprofilbeschreibung (Textbausteine werden basierend auf nur neun Hauptcodes der Codeauswahl 10.1 generiert), Schritt S3.3 (zusammengefasste Persönlichkeitsprofilbeschreibung insbesondre basierend auf neun Hauptcodes). Die überblickartige Persönlichkeitsprofilbeschreibung 10i lässt sich vorteilhaft auf z.B. nur einer halben DinA4-Seite konsumieren bzw. kann auch computergestützt vorgelesen werden, und/oder visualisiert ausgegeben werden, insbesondere als visualisierte 3x3-Matrix, wie beispielhaft in Fig. 9 verdeutlicht. Eine entsprechende Vorgabe für den Umfang und/oder die Art und Weise der Ausgabe kann der jeweilige Nutzer auch über die in Fig. 2 beschriebenen Felder 23 der Benutzerschnittstelle vornehmen. Wahlweise kann dabei auch ein ergänzendes Feld speziell für die grafische 3x3-Visualisierung vorgesehen sein.

In **Fig. 9** ist eine grafische Visualisierung gezeigt, welche die in den basierend auf den generierten Codes erstellten Textblöcken enthaltene Information noch leichter erfassbar machen; diese Art 3x3-Matrix kann wahlweise ergänzend zu einer textbasierten Beschreibung ausgegeben werden, insbesondere basierend auf den hier beschriebenen neun (9) codesetübergreifenden Hauptcodes. Vorteilhaft wird dabei ein die mentale Ebene betreffender Code 10.11 bzw. dessen Textbaustein-Pendant (...) in einer obersten Ebene der 3x3-Matrix dargestellt, und ein die emotionale Ebene betreffender Code 10.11 bzw. dessen Textbaustein-Pendant (...) in einer mittleren Ebene der 3x3-Matrix dargestellt, und ein die körperliche Ebene betreffender Code 10.11 bzw. dessen Textbaustein-Pendant (...) in einer untersten Ebene der 3x3-Matrix dargestellt. Vorteilhaft wird dabei ein die transzendente Ebene betreffender Code 10.11 bzw. dessen Textbaustein-Pendant (...) in einer mittleren vertikalen Ebene der 3x3-Matrix dargestellt.

### Bezugszeichenliste

- 1: Datenbank
- 2: Server
- 3: Charakterprofildatensatz für jeweils ein Charakterprofil
- 3a: Human Design-Charakterprofildatensatz
- 3b: Genekeys-Charakterprofildatensatz
- 3c: Bazi Suan Ming-Charakterprofildatensatz
- 3d: Numerologie-Charakterprofildatensatz
- 3e: Astrologie-Charakterprofildatensatz
- 3.1: eigenständiger Codeset eines spezifischen Charakterprofildatensatzes
- 3.11: einzelner Code, korrelierbar/korreliert mit einzelnem Charaktermerkmal
- 10: Persönlichkeitsprofildatensatz, insbesondere in Matrixkonfiguration
- 10i: codesetübergreifendes Persönlichkeitsprofil
- 10i': codesetübergreifendes gewichtetes Persönlichkeitsprofil mit Scoring
- 10.1: Codeauswahl aus der Mehrzahl von eigenständigen Codesets
- 10.11: codesetübergreifender Persönlichkeitseigenschafts-Code
- 14: Attribut (Priorisierungs-/Gewichtungsparameter)
- 20: Benutzerschnittstelle
- 21: Eingabefeld für individuenspezifischen Kennwert
- 22: Eingabefeld für Attribut
- 23: Eingabefeld für Detaillierungsgrad (kurz/detailliert)
- 25: Ausgabemittel (visuell und/oder akustisch)
- 100: Endgerät
- i: individuenspezifischer Kennwert, insbesondere Geburtsdatum, Geburtszeit, Geburtsort
- I: Individuum
- B: Profilbild (frontal)
- S1: Schritt Dateneingabe insbesondere über Benutzerschnittstelle
- S2: Schritt Erstellung Datengrundlage: Profilbestimmung basierend auf mehreren Ansätzen
- S2.1: Schritt Erstellung Datengrundlage: Profilbestimmung basierend auf Human Design-Ansatz
- S2.2: Schritt Erstellung Datengrundlage: Profilbestimmung basierend auf Genekeys-Ansatz
- S2.3: Schritt Erstellung Datengrundlage: Profilbestimmung basierend auf Bazi Suan Ming-Ansatz
- S2.4: Schritt Erstellung Datengrundlage: Profilbestimmung basierend auf Numerologie-Ansatz
- S2.5: Schritt Erstellung Datengrundlage: Profilbestimmung basierend auf Astrologie-Ansatz
- S2.x: charakterprofildatensatzspezifischer Codeoutput
- S3: Schritt Auswertung Datengrundlage aus der Mehrzahl von Ansätzen
- S3.1: Korrelieren von Textbausteinen mit Codes
- S3.2: Ausgabe eines/des individuenspezifischen Persönlichkeitsprofils insbesondere als (Fließ-)Text
- S3.3: zusammengefasste Persönlichkeitsprofilbeschreibung basierend auf neun Hauptcodes
- S3.x: charakterprofildatensatzübergreifender Codeoutput
- S2S3: iterative Codegenerierung
- S4: Schritt Attributieren: Erstellen/Berücksichtigen von Attributen
- S5: Schritt Abgleichen (Matching) von Persönlichkeitsprofilen
- S6: Schritt Ausgabe von Gruppen zueinander korrelierter/korrelierbarer Individuen
- S6.1: Identifizierung eines Individuums als "geeignet", wahlweise Zuweisung zu einer Gruppe
- S6.2: Identifizierung eines Individuums als "ungeeignet"
- S7: computergestütztes/-implementiertes Auswählen/Kontaktieren wenigstens eines Individuums

## Patentansprüche

1. Verfahren zum computergestützten/-implementierten Erstellen eines Persönlichkeitsprofils eines Individuums (I) und zum Abgleichen des Persönlichkeitsprofils mit dem Persönlichkeitsprofil wenigstens eines weiteren Individuums, wobei eine Datengrundlage für die Erstellung des Persönlichkeitsprofils basierend auf zumindest den folgenden über eine Benutzerschnittstelle (20) manuell eingebbaren oder zumindest teilweise automatisiert aus einer Datenbank (1) abrufbaren individuenspezifischen Kennwerten (i) erstellt oder abgerufen sowie ausgewertet wird: Geburtsdatum, Geburtszeit, Geburtsort;
**dadurch gekennzeichnet, dass** die Datengrundlage des Persönlichkeitsprofils computergestützt/-implementiert weiter ergänzt wird, indem eine Mehrzahl von eigenständigen Charakterprofildatensätzen (3) zum individuenspezifischen Erstellen/Generieren/Auswerten eines/des charakterprofildatensatzübergreifenden Persönlichkeitsprofils (10i) herangezogen und ausgewertet werden, insbesondere wenigstens fünf eigenständige Charakterprofildatensätze (3), insbesondere Charakterprofildatensätze betreffend die folgenden Charakterprofile: Human Design-Profildaten, Genekeys-Profildaten, Numerologie-Profildaten, Bazi Suan Ming-Profildaten, Astrologie-Profildaten, wobei aus der Datengrundlage eine Mehrzahl von codesetübergreifenden Persönlichkeitseigenschafts-Codes erzeugt werden, indem eine Mehrzahl von eigenständigen Codesets, insbesondere wenigstens fünf eigenständige Codesets genutzt werden.

2. Verfahren nach dem vorhergehenden Verfahrensanspruch, wobei wenigstens einer der eigenständigen Charakterprofildatensätze auf dem Yijing basiert und eine Mehrzahl von Codes basierend auf den 64 Hexagrammen des Yijing bereitstellt, aus welchen eine Codeauswahl für ein Codeset getroffen wird, welcher den entsprechenden eigenständigen Charakterprofildatensatz charakterisiert und welcher eingerichtet ist, mit wenigstens einem Codeset eines der weiteren eigenständigen Charakterprofildatensätze korreliert zu werden; und/oder wobei wenigstens vier der eigenständigen Charakterprofildatensätze auf dem Yijing basieren und eine Mehrzahl von Codes jeweils basierend auf den 64 Hexagrammen des Yijing bereitstellen, wobei diese Codes mit Codes des Numerologie-Ansatzes kombiniert werden, zum Bilden der codesetübergreifenden Persönlichkeitseigenschafts-Codes.

3. Verfahren nach einem der vorhergehenden Verfahrensansprüche, wobei vier der eigenständigen Charakterprofildatensätze auf dem Yijing basieren, wobei aus jeweils einem dieser Charakterprofildatensätze Codes betreffend eine der folgenden Charakterebenen entnommen oder ausgewählt werden: transzendente Charakterebene, emotionale Charakterebene, körperliche Charakterebene, mentalen Charakterebene; und/oder wobei vier der eigenständigen Charakterprofildatensätze auf dem Yijing basieren, wobei ein erster Codeset aus dem Human Design-Ansatz und ein zweiter Codeset aus dem Genekeys-Ansatz und ein dritter Codeset aus dem Bazi Suan Ming-Ansatz generiert oder verwendet werden, zum Bilden eines Codeset, welcher mit einem vierten Codeset aus dem Astrologie-Ansatz korreliert wird, und wobei ein weiterer Codeset aus dem Numerologie-Ansatz generiert oder verwendet wird, zum Bilden der codesetübergreifenden Persönlichkeitseigenschafts-Codes.

4. Verfahren nach einem der vorhergehenden Verfahrensansprüche, wobei vier der eigenständigen Charakterprofildatensätze auf dem Yijing basieren, wobei ein erster Charakterprofildatensatz auf dem Human Design-Ansatz basiert und einen ersten Codeset liefert, welcher eine mentale Charakterebene beschreibt, wobei ein zweiter Charakterprofildatensatz auf dem Genekeys-Ansatz basiert und einen zweiten Codeset liefert, welcher eine emotionale Charakterebene beschreibt, wobei ein dritter Charakterprofildatensatz auf dem Bazi Suan Ming-Ansatz basiert und einen dritten Codeset liefert, welcher eine transzendente Charakterebene beschreibt, wobei ein vierter Charakterprofildatensatz auf dem Numerologie-Ansatz basiert und einen vierten Codeset liefert, welcher eine körperliche Charakterebene beschreibt; und/oder wobei zunächst drei eigenständige Charakterprofildatensätze, die jeweils auf dem Yijing basieren, miteinander kombiniert werden, zum Bilden von ersten codesetübergreifenden Codes, und daraufhin werden ein/der auf dem Numerologie-Ansatz basierende Charakterprofildatensatz und ein/der auf dem Astrologie-Ansatz basierende Charakterprofildatensatz kombiniert, zum Bilden von zweiten codesetübergreifenden Codes, wobei das Persönlichkeitsprofil aus den ersten und zweiten codesetübergreifenden Codes generiert wird.

5. Verfahren nach einem der vorhergehenden Verfahrensansprüche, wobei vier der eigenständigen Charakterprofildatensätze auf dem Yijing basieren und jeweils ein Codeset liefern, wobei diese vier Codesets zu einem korrelierten Codeset zusammengeführt werden, wobei ein weiterer eigenständiger Charakterprofildatensatz auf einem/dem Numerologie-Ansatz basiert und einen weiteren Codeset liefert, welcher mit dem korrelierten Codeset der vier auf dem Yijing basierenden Charakterprofildatensätze kombiniert wird; oder wobei vier der eigenständigen Charakterprofildatensätze auf dem Yijing basieren und jeweils ein Codeset liefern, wobei ein weiterer eigenständiger Charakterprofildatensatz auf einem/dem Numerologie-Ansatz basiert und einen weiteren Codeset liefert, welcher mit den Codesets der vier auf dem Yijing basierenden Charakterprofildatensätze kombiniert wird; und/oder wobei eine Mehrzahl der eigenständigen Charakterprofildatensätze auf dem Yijing basieren, wobei ein erster Charakterprofildatensatz auf dem Human Design-Ansatz basiert und einen ersten Codeset liefert, welcher eine mentale Charakterebene beschreibt, wobei aus diesem Codeset alle verfügbare Codes genutzt werden, und wobei aus wenigstens einem der weiteren eigenständigen Charakterprofildatensätzen jeweils nur eine Codeauswahl generiert wird.

6. Verfahren nach einem der vorhergehenden Verfahrensansprüche, wobei 64 codesetübergreifende Persönlichkeitseigenschafts-Codes generiert werden; und/oder wobei 64 codesetübergreifende Persönlichkeitseigenschafts-Codes generiert werden, aus denen neun Codes als oberbegriffsartige Hauptcodes definiert sind/werden, basierend auf welchen eine Ausgabe des Persönlichkeitsprofils im Umfang eines Fließtextes von ca. einer halben DinA4-Seite, maximal einer ganzen DinA4-Seite erfolgt; und/oder wobei für jedes Individuum alle ermittelten oder verfügbaren Codes bestimmt oder ausgelesen werden und mit allen ermittelten oder verfügbaren Codes des wenigstens einen weiteren Individuums basierend auf den jeweils daraus individuenspezifisch generierten codesetübergreifenden Persönlichkeitseigenschafts-Codes verglichen/abgeglichen werden, wobei eine Zuweisung von Individuen zu derselben Gruppe bevorzugt basierend auf wenigstens einem abgleichspezifisch vorgebbaren Attribut zum Einstellen des Impact eines jeweiligen Persönlichkeitseigenschafts-Codes erfolgt.

7. Verfahren nach einem der vorhergehenden Verfahrensansprüche, wobei aus der Datengrundlage die Mehrzahl von codesetübergreifenden Persönlichkeitseigenschafts-Codes erzeugt werden, indem wenigstens fünf eigenständige Codesets genutzt werden, nämlich Codesets betreffend die folgenden Charakterprofildatensätze: Human Design-Profildaten, Genekeys-Profildaten, Numerologie-Profildaten, Bazi Suan Ming-Profildaten, astrologische Profildaten; und/oder wobei für ein spezifisches Individuum mehrere Codes eigenständiger Codesets miteinander kombiniert werden zum Erweitern der individuenspezifischen Datengrundlage durch daraus generierte codesetübergreifende Persönlichkeitseigenschafts-Codes; und/oder wobei die Datengrundlage des Persönlichkeitsprofils computergestützt/-implementiert weiter ergänzt wird, indem wenigstens fünf eigenständige Charakterprofildatensätze zum individuenspezifischen Erstellen/Generieren/Auswerten eines/des charakterprofildatensatzübergreifenden Persönlichkeitsprofils herangezogen und ausgewertet werden, nämlich wenigstens die fünf folgenden eigenständigen Charakterprofildatensätze: Human Design, Genekeys, Bazi Suan Ming, Numerologie, Astrologie; insbesondere in der hier genannten Reihenfolge; und/oder wobei aus wenigstens fünf eigenständigen Charakterprofildatensätzen die folgende Codeauswahl getroffen wird: Human Design-Codes in der gesamten Verfügbarkeit, dreizehn Genekeys-Codes, zehn Bazi Suan Ming-Codes, zwanzig Numerologie-Codes, Astrologie-Codes in der gesamten Verfügbarkeit.

8. Verfahren nach einem der vorhergehenden Verfahrensansprüche, wobei jeder codesetübergreifende Persönlichkeitseigenschafts-Code mit einem einzigen Textbaustein korreliert ist/wird, wobei das Persönlichkeitsprofil in Ausgestaltung als lesbarer Text computerautomatisiert generiert wird basierend auf der Gesamtheit der individuenspezifisch ermittelten codesetübergreifenden Persönlichkeitseigenschafts-Codes; und/oder wobei zumindest die folgenden individuenspezifischen Kennwerte über eine Benutzerschnittstelle eingebbar sind und daraufhin computerautomatisiert auswertbar sind, insbesondere zur Datenpflege einer/der durch die jeweils individuenspezifisch verwirklichten codesetübergreifenden Persönlichkeitseigenschafts-Codes gebildeten Matrix: Geburtsdatum, Geburtszeit, Geburtsort; und/oder wobei für den Abgleich der Persönlichkeitsprofile wenigstens ein Attribut vorgebbar ist, über welches die Relevanz eines jeweiligen individuenspezifisch ermittelten codesetübergreifenden Persönlichkeitseigenschafts-Codes vordefiniert/vordefinierbar ist.

9. Verfahren nach einem der vorhergehenden Verfahrensansprüche, wobei der Abgleich der Persönlichkeitsprofile oder Persönlichkeitseigenschaften von wenigstens zwei Individuen basierend auf einem mittels der individuenspezifisch ermittelten codesetübergreifenden Persönlichkeitseigenschafts-Codes und wenigstens eines vorgebbaren Attributs erstellten anwendungsspezifisch gewichteten Klassifizierung erfolgt, wobei die jeweils wenigstens zwei miteinander verglichenen Individuen basierend auf einer wenigstens drei- oder vierstufigen Klassifizierung, welche die Anzahl der ermittelten übereinstimmenden Persönlichkeitseigenschaften abbildet, entweder einer gleichen Gruppe von Individuen zugeordnet werden oder jeweils einer anderen Gruppe von Individuen zugewiesen oder als nicht miteinander vereinbar klassifiziert werden: 90-100% Vereinbarkeit/Zuweisung, 75-89% Vereinbarkeit/Zuweisung potentiell möglich, <75% Vereinbarkeit/Zuweisung potentiell zu verneinen, und optional <50% Zuweisung unmöglich; und/oder wobei ein Abgleich der Persönlichkeitsprofile oder Persönlichkeitseigenschaften von wenigstens drei Individuen erfolgt, wobei wenigstens eines der abgeglichenen Individuen basierend auf einer mittels wenigstens eines anwendungsspezifisch vorgegebenen Attributs erstellten gewichteten Klassifizierung gegenüber wenigstens einem der anderen Individuen priorisiert zugewiesen wird, insbesondere auch dann wenn die Anzahl korrelierender individuenspezifisch ermittelter codesetübergreifender Persönlichkeitseigenschafts-Codes gleich groß ist; und/oder wobei für den Abgleich von wenigstens zwei Persönlichkeitsprofilen wenigstens ein Attribut vorgegeben wird, über welches die Relevanz eines jeweiligen codesetübergreifenden Persönlichkeitseigenschafts-Codes für den jeweiligen Abgleich der Persönlichkeitsprofile vordefiniert wird.

10. Computerprogrammprodukt umfassend Befehle, die bei Ausführung des Computerprogrammproduktes auf einem Computer diesen dazu veranlassen, ein Verfahren nach einem der vorhergehenden Verfahrensansprüche auf dem Computer auszuführen; wobei das Computerprogramm bevorzugt auch eingerichtet ist zum Abgleichen der Persönlichkeitsprofile (10i) von wenigstens zwei Individuen (I) unter Bezugnahme sowohl auf die ersten als auch auf die zweiten Codes des jeweiligen Individuums.

11. Datenbank (1) mit wenigstens einer darin hinterlegten und computerautomatisiert aufrufbaren und abgleichbaren Matrix, in welcher individuenspezifisch eine Vielzahl von Codes (3.11) jeweils charakterisierend eine einzige Persönlichkeitseigenschaft hinterlegt sind, wobei die Datenbank (1) eingerichtet ist zur Implementierung eines Verfahren nach einem der Verfahrensansprüche 1 bis 10 zum Abgleichen der Persönlichkeitseigenschaften und zum Generieren von Gruppenempfehlungen betreffend miteinander harmonisierende Individuen (I), insbesondere mittels eines Computerprogrammproduktes nach Anspruch 10, insbesondere basierend auf vordefinierbaren Attributen betreffend wenigstens eine Persönlichkeitseigenschaft.

12. Benutzerschnittstelle (20) mit einer Anzeige-/Bedienmaske eingerichtet zum Eingeben wenigstens eines individuenspezifischen Kennwerts (i) zum Erstellen eines Persönlichkeitsprofils (10i) wenigstens eines Individuums (I), insbesondere zum Abgleichen des Persönlichkeitsprofils mit dem Persönlichkeitsprofil wenigstens eines weiteren Individuums, wobei die Benutzerschnittstelle (20) eingerichtet ist zum Einpflegen zumindest der folgenden individuenspezifischen Kennwerte (i):
Geburtsdatum, Geburtszeit, Geburtsort;
**dadurch gekennzeichnet, dass** die Benutzerschnittstelle (20) ferner eingerichtet ist zum Ausgeben einer Mehrzahl von codesetübergreifenden Persönlichkeitseigenschafts-Codes (10.11) für das jeweilige Individuum (I), insbesondere mit den Codes in übersetzter Ausgestaltung als durch das Individuum lesbare Textbausteine, wobei die Persönlichkeitseigenschafts-Codes unter Bezugnahme auf eine Mehrzahl von eigenständigen Charakterprofildatensätzen (3) erzeugt sind, insbesondere gemäß einem Verfahren gemäß einem der Ansprüche 1 bis 9, insbesondere unter Bezugnahme auf wenigstens fünf eigenständige Charakterprofildatensätze, insbesondere Charakterprofildatensätze betreffend die folgenden Charakterprofile: Human Design-Profildaten, Genekeys-Profildaten, Numerologie-Profildaten, Bazi Suan Ming-Profildaten, Astrologie-Profildaten, wobei die Mehrzahl von codesetübergreifenden Persönlichkeitseigenschafts-Codes aus einer Mehrzahl von eigenständigen Codesets, insbesondere wenigstens fünf eigenständige Codesets gebildet ist/wird.

13. Benutzerschnittstelle nach Anspruch 12, wobei die Benutzerschnittstelle ferner eingerichtet ist zum Ausgeben einer Auswahl wenigstens eines Individuums aus einer Gruppe von Individuen, bezüglich welcher die Codes zum Abgleich der Persönlichkeitsprofile (10i) der Individuen verglichen wurden; und/oder wobei die Benutzerschnittstelle eingerichtet ist zum Abfragen wenigstens eines wenigstens eine Persönlichkeitseigenschaft priorisierenden Attributs; und/oder wobei die Benutzerschnittstelle eingerichtet ist zum Abfragen eines Gewichtungsfaktors wenigstens eines wenigstens eine Persönlichkeitseigenschaft priorisierenden Attributs; und/oder wobei die Benutzerschnittstelle eingerichtet ist zum Abfragen eines Schwellwerts hinsichtlich eines Grades einer Übereinstimmung von codesetübergreifenden Codes; und/oder wobei die Benutzerschnittstelle eingerichtet ist zum Einpflegen und Ausgeben eines Profilbildes und/oder eines Namens des jeweiligen Individuums; und/oder wobei die Benutzerschnittstelle eingerichtet ist zum Ausgeben einer Team-Zusammensetzung umfassend eine Vielzahl von Individuen mit jeweils aufeinander abgestimmten Persönlichkeitsprofilen, insbesondere in Abhängigkeit von vordefinierbaren über die Benutzerschnittstelle eingebbaren Attributen für die aufeinander abzustimmenden Persönlichkeitsprofile; und/oder wobei die Benutzerschnittstelle ferner eingerichtet ist zum Visualisieren einer systemseitig basierend auf dem Abgleich der Persönlichkeitsprofile empfohlenen Gruppenzusammensetzung aus wenigstens drei Individuen.

14. Benutzerschnittstelle nach einem der Ansprüche 12 oder 13, wobei die Benutzerschnittstelle eingerichtet ist zum Ausgeben von einer Auswahl von neun Persönlichkeitseigenschafts-Hauptcodes in einer 3x3-Visualisierung, insbesondere gemäß einem Puzzle mit neun Teilen oder gemäß einer 3x3-Martix, insbesondere bei farblicher Hervorhebung, insbesondere mit einer/der Darstellung der mentalen Ebene oberhalb von einer/der Darstellung der emotionalen Ebene oberhalb von einer/der Darstellung der körperlichen Ebene, insbesondere mit einer/der Darstellung der transzendenten Ebene im vertikal-mittigen Bereich; und/oder wobei die Benutzerschnittstelle eingerichtet ist zum Ausgeben der Information von 64 codesetübergreifenden Persönlichkeitseigenschafts-Codes auf wenigstens zwei unterschiedlichen Detaillierungsstufen, insbesondere über ein spezifisches Eingabefeld für den Detaillierungsgrad, von denen neun Codes als oberbegriffsartige Hauptcodes definiert sind, basierend auf welchen eine Ausgabe des Persönlichkeitsprofils im Umfang eines Fließtextes von ca. einer halben DinA4-Seite, maximal einer ganzen DinA4-Seite realisierbar ist.

15. Verwendung einer in einer Datenbank (1) hinterlegten Matrix mit individuenspezifisch hinterlegten codesetübergreifenden Persönlichkeitseigenschafts-Codes (10.11) jeweils charakterisierend eine Persönlichkeitseigenschaft zum computerautomatisierten/-implementierten Aufrufen und Abgleichen von Persönlichkeitsprofilen (10i) von einer Vielzahl von Individuen (I) hinsichtlich wenigstens eines vordefinierbaren Soll-Persönlichkeitsprofils, wobei ein Abgleich von zumindest den folgenden Persönlichkeitseigenschafts-Codes (10.11) erfolgt: Human Design-Codes, Genekeys-Codes, Numerologie-Codes, Bazi Suan Ming-Codes, Astrologie-Codes; insbesondere mit dem Soll-Persönlichkeitsprofil erstellt in Abhängigkeit von den Persönlichkeitseigenschaften einer Mehrzahl von Persönlichkeitsprofilen von Individuen einer bereits angelegten Gruppe von Individuen mit jeweils individuell individuenspezifisch vordefiniertem Soll-Persönlichkeitsprofil, insbesondere unter Anwendung eines Verfahrens gemäß einem der Verfahrensansprüche 1 bis 9, wobei die Mehrzahl von codesetübergreifenden Persönlichkeitseigenschafts-Codes aus einer Mehrzahl von eigenständigen Codesets, insbesondere wenigstens fünf eigenständige Codesets gebildet ist/wird.
